(19) Europäisches Patentamt
European Patent Office
Office européen des brevets

(11) EP 0 489 647 B1

(12) **FASCICULE DE BREVET EUROPEEN**

(45) Date de publication et mention de la délivrance du brevet:
**12.03.1997 Bulletin 1997/11**

(51) Int Cl.[6]: **C08B 37/00**, A61K 31/725

(21) Numéro de dépôt: **91403272.7**

(22) Date de dépôt: **03.12.1991**

(54) **Héparosanes-N,O-sulfatés, leur procédé de préparation et les compositions pharmaceutiques qui les contiennent**

N,O-sulfatierte Heparosane; Verfahren zu deren Herstellung und diese enthaltende Arzneimittel

N,O-sulfated heparosanes; process to produce the same and pharmaceutical compositions containing them

(84) Etats contractants désignés:
**AT BE CH DE DK ES FR GB GR IT LI LU MC NL SE**

(30) Priorité: **03.12.1990 FR 9015114**

(43) Date de publication de la demande:
**10.06.1992 Bulletin 1992/24**

(83) **Déclaration conformément à la règle 28(4) CBE (solution de l'expert)**

(73) Titulaire: **SANOFI**
**75008 Paris (FR)**

(72) Inventeurs:
- **Lormeau, Jean Claude**
**F-94270 Kremlin Bicêtre (FR)**
- **Chevallier, Bruno**
**F-75013 Paris (FR)**
- **Salome, Marc Louis Victor**
**F-31320 Castanet-Tolosan (FR)**
- **Tenaille d'Estais, Guy Etienne Marie**
**F-31400 Toulouse (FR)**

(74) Mandataire: **Le Guen, Gérard et al**
**CABINET LAVOIX**
**2, place d'Estienne d'Orves**
**75441 Paris Cédex 09 (FR)**

(56) Documents cités:
**EP-A- 0 116 801** **EP-A- 0 287 477**
**EP-A- 0 333 243** **FR-A- 2 584 728**

- **ANALYTICAL BIOCHEMISTRY vol. 135, 1983, pages 134 - 140; J. L. NAVIA ET AL: 'Assay of N-acetylheparosan deacetylase with a capsular polysaccharide from Escherichia coli K5 as substrate'**
- **W.F.Vann et al., European Journal of Biochemistry, vol. 116 (1981), pages 359-364**

Remarques:
Le dossier contient des informations techniques présentées postérieurement au dépôt de la demande et ne figurant pas dans le présent fascicule.

EP 0 489 647 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention a pour objet de nouveaux héparosanes-N,O-sulfatés, les compositions d'héparosanes-N,O-sulfatés contenant ces nouveaux héparosanes-N,O-sulfatés, et les compositions pharmaceutiques ayant comme principe actif les nouveaux héparosanes-N,O-sulfatés.

Il est connu que les glycosaminoglycanes sont des produits susceptibles d'être obtenus par extraction des tissus animaux. Certains de ces glycosaminoglycanes possèdent des propriétés anticoagulantes et antithrombotiques très intéressantes. Des produits typiques de cette famille sont l'héparine, ses fragments et leurs dérivés, ainsi que l'héparane-sulfate et le dermatane-sulfate, qui ont cependant le désavantage, dû à leur origine, d'être très coûteux.

En particulier, il est connu que le dermatane-sulfate est une famille de polymères de degré de polymérisation variable, formées d'unités répétées constituées d'un groupe acide uronique (iduronyle ou glucuronyle) et d'un groupe acétylsulfo-4-galactosaminyle (H.W. Stuhlsatz "The Methodology of Connective Tissue Research", (1976), 137-146). Le dermatane-sulfate naturel a une masse moléculaire comprise entre 20.000 et 40.000 Da. Ce produit est particulièrement intéressant comme anticoagulant et antithrombotique (F. Fernandez et al, British Journal of Haematology, (1986), 64, 309-317).

Il est d'autre part connu (I. Björk et U. Lindahl, "Molecular and Cellular Biochemistry", (1982), Dr. W. Junk Publishers - Pays-Bas) que la coagulation sanguine est un phénomène physiologique complexe, dont le mécanisme peut être schématisé et résumé de la façon suivante :

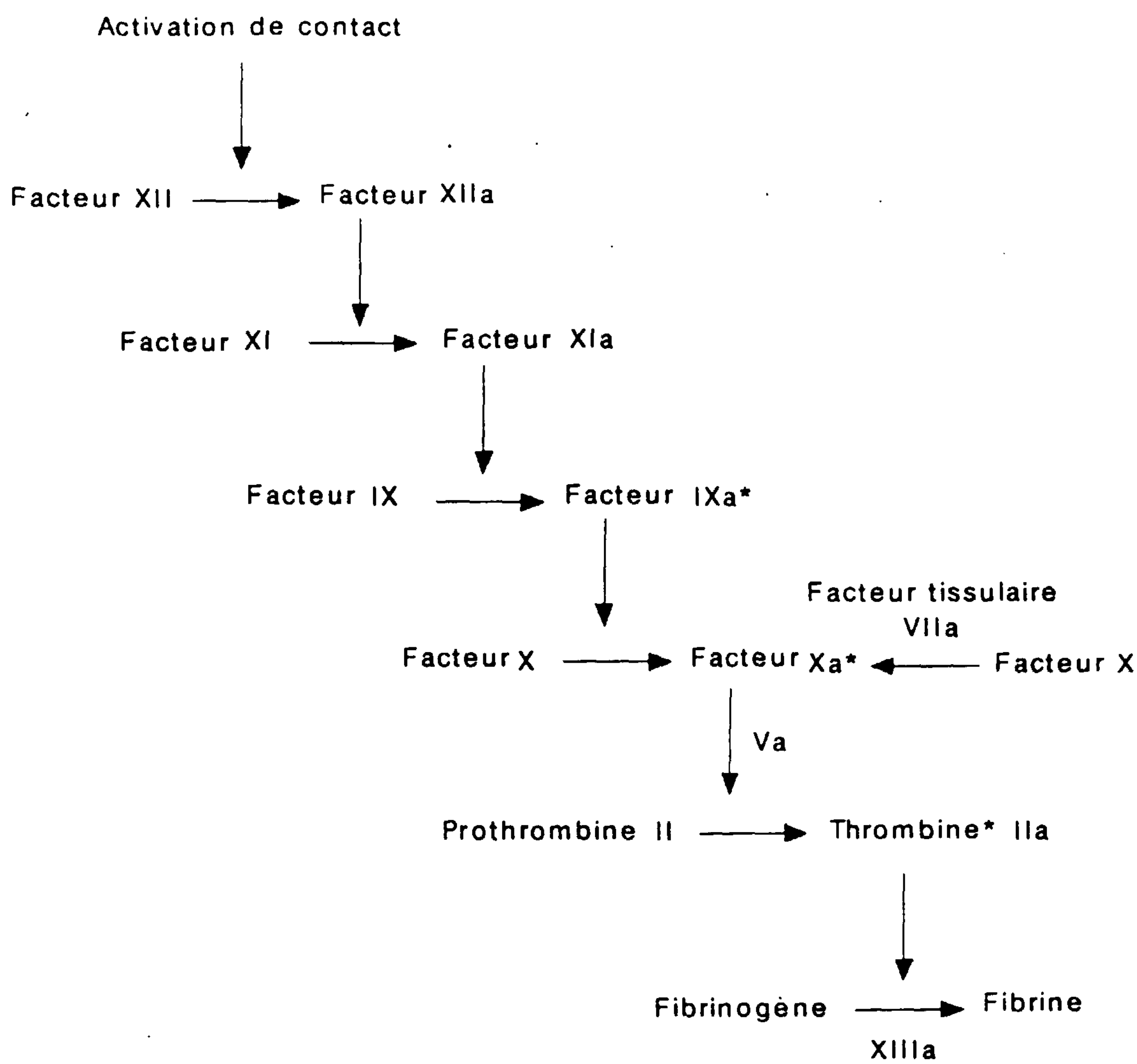


Certains stimuli, tels que l'activation de contact et les facteurs tissulaires, déclenchent l'activation successive d'une

série de facteurs de coagulation présents dans le plasma sanguin, ceux-ci étant repérés par des chiffres romains sur le schéma ci-dessus et la présence de l'indice a désignant la forme activée (indice a présent) ou non activée (indice a absent) d'un facteur de coagulation donné.

Quelle que soit la nature du stimulus, les étapes finales sont identiques : le facteur Xa active le facteur II (également appelé prothrombine), lequel sous sa forme activée (facteur IIa également appelé thrombine) provoque la protéolyse partielle du fibrinogène soluble avec libération de la fibrine insoluble, constituant principal du caillot sanguin.

Dans les conditions physiologiques normales, des protéines régulatrices telles que l'antithrombine III (ATIII) et le cofacteur II de l'héparine (HCII), sont également présentes dans le plasma.

L'antithrombine III exerce une activité inhibitrice sur l'ensemble des facteurs de coagulation repérés par un astérisque (*) dans le schéma ci-dessus. Cette inhibition est amplifiée de façon très forte en présence d'héparine ou d'oligosaccharides de synthèse de type héparinique (D.H. Atha et al, Biochemistry, (1985), 24, 6723-6729).

Le cofacteur II de l'héparine n'exerce une activité inhibitrice que sur le facteur IIa (thrombine), lequel catalyse la dernière étape de la coagulation. Cette activité est amplifiée de façon importante en présence d'héparine ou de dermatane-sulfate (D.M. Tollefsen, J. Biol. Chem, (1983), 258, 6713-6716).

L'inhibition du facteur Xa ou du facteur IIa constitue un moyen privilégié pour obtenir une activité anticoagulante et antithrombotique puisque ces deux facteurs interviennent dans les deux dernières étapes de la coagulation, lesquelles sont indépendantes du stimulus déclencheur.

Pour obtenir l'inhibition du facteur IIa seul, une possibilité particulièrement intéressante consiste à mettre à profit la spécificité du cofacteur II de l'héparine et à rechercher l'amplification de son activité inhibitrice. Le dermatane-sulfate est le produit connu possédant l'activité amplificatrice de ce type la plus puissante.

Il est également connu que la constitution de la chaîne héparinique principale se fait en deux étapes. Dans un premier temps, l'héparine est biosynthétisée à partir d'un protéoglycane précurseur, dont la partie polysaccharidique est constituée par une famille de polymères de degré de polymérisation variable formés d'unités répétées β-D-glucuronyl-1,4-α-N-acétyl-D-glucosaminyl-(1,4) (unités disaccharidiques) de masse moléculaire 379 Da. Cette partie polysaccharidique est habituellement appelée N-acétylhéparosane (J. Navia, Anal. Biochem., (1983), 135, 134-140). Cette première étape de biosynthèse est le seul moment où l'on peut vraiment parler d'un "motif disaccharidique" car la seconde étape de la biosynthèse va modifier ce squelette simple d'une manière profonde (" L'héparine, fabrication, structure, propriétés, analyses", J.P. Duclos, (1984) pp 81-83, Masson Ed.-France).

En effet, l'héparine naturelle, résultat de la biosynthèse est un polysaccharide constitué par des molécules d'acide glucuronique et d'acide iduronique (acides uroniques), éventuellement sulfaté en position 2, associé à des molécules de glucosamine, éventuellement sulfatée en position 6 et sulfatée ou acétylée sur l'amine en position 2.

La structure de l'héparine peut-être statistiquement représentée par la formule (i) suivante :

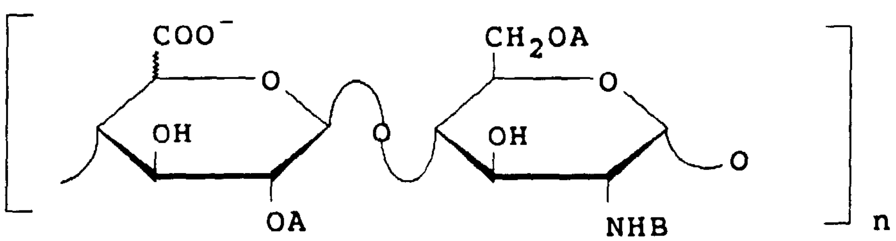

(i)

où A représente H et $SO_3^-$, B représente $SO_3^-$ et $COCH_3$ et n est un nombre entier compris entre 20 et 30, ce qui correspond à un poids moléculaire de 12000 à 18000 Da (EP-A-0 116 801 ).

Les expressions "H et $SO_3^-$" et "$SO_3^-$ et $COCH_3$" telles que respectivement utilisées pour les substituants A et B, indiquent que dans les 20 à 30 unités disaccharidiques ci-dessus, A est dans certains cas l'hydrogène et dans d'autres cas un groupe $SO_3^-$ , et, d'une façon analogue, B est dans la majorité des cas $SO_3^-$ et dans d'autres cas un groupe acétyle.

De même, la liaison ,⌇ signifie que le groupe $COO^-$, dans les 20 à 30 unités disaccharidiques ci-dessus, a dans certains cas la configuration (ii) :

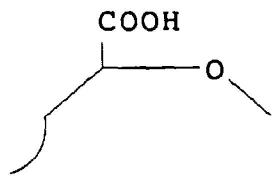

(ii)

(il s'agit de l'acide D-glucuronique)

et dans la majorité des n unités, la configuration (iii) :

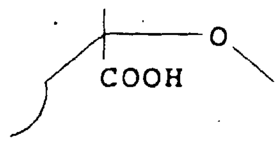

(iii)

(il s'agit de l'acide L-iduronique).

Donc, l'héparine et les fragments d'héparine tels qu'obtenus par différentes méthodes de dépolymérisation, sont des macromolécules contenant aussi bien des unités d'acide glucuronique que des unités d'acide iduronique.

Certaines méthodes de dépolymérisation permettent d'obtenir des fragments d'héparine ayant un poids moléculaire compris entre 2000 et 9000 Da et un degré de sulfatation supérieur d'au moins 20 % à celui de l'héparine de départ. De telles héparines "supersulfatées" sont décrites dans la demande de brevet EP-A-0 116 801, et possèdent pour les unités uroniques, les deux structures (ii) et (iii) mentionnées ci-dessus.

Il est aussi connu que certaines bactéries de l'espèce Escherichia coli produisent un polysaccharide capsulaire, habituellement appelé antigène K5, qui est une famille de polymères constituée d'unités répétées β-D-glucuronyl-1,4-α-N-acétyl-D-glucosaminyl-(1,4), (W.F. Vann et al, Eur. J. Biochem, (1981), 116, 359-364).

Ce polysaccharide, de même nature chimique que la partie polysaccharidique du protéoglycane précurseur de l'héparine, sera appelé ici N-acétylhéparosane. Ce produit possède une masse moléculaire comprise entre $10^5$ et $2,0.10^6$ Da, et au niveau des unités "acide uronique", une structure très régulière composée uniquement d'acide D-glucuronique (W.F. Vann et al, Eur. J. Biochem., (1981), 116, 359-364, et demande de brevet EP-A-0 333 243).

La demande de brevet EP-A-0 333 243 décrit le polysaccharide K5 O-sulfaté ainsi que certains de ses fragments composés respectivement de 4, 6 ou 8 unités "sucres", également O-sulfatés. Ces produits ont une activité antiangiogénique et antitumorale, avec un rapport favorable de ces activités par rapport aux propriétés anticoagulantes. Ce document décrit également des fragments de N-acétylhéparosane composés respectivement de 4, 6, 8 ou 10 unités "sucres".

La demande de brevet EP-A-0 333 243 décrit aussi la préparation d'un pentasaccharide ayant la structure N-acétylhéparosane-O-sulfaté, par synthèse chimique totale.

On a maintenant trouvé que les héparosanes-N,O-sulfatés de poids moléculaire variable compris entre 1500 et 15000 Da possèdent une activité anticoagulante vis à vis de l'héparine cofacteur II et une activité anti-Xa très élevée.

Les héparosanes-N,O-sulfatés, objet de la présente invention, se distinguent donc des autres produits déjà décrits dans la littérature, par leur structure originale, notamment leur degré de sulfatation (sulfatation aussi sur le groupe amine de la glucosamine) et leurs propriétés pharmacologiques. Ils possèdent parmi d'autres propriétés pharmacologiques, une activité anticoagulante vis à vis de l'héparine cofacteur II (HCII) plus forte que celle du dermatane-sulfate et ils ont des caractéristiques pharmacocinétiques très intéressantes. En effet, les produits de l'invention qui sont des produits obtenus par hémisynthèse chimique, possèdent les activités pharmacologiques des glycosaminoglycanes couramment utilisés en thérapeutiques, notamment celles de l'héparine, et peuvent être utiles pour la régulation de la coagulation et peuvent plus particulièrement trouver leurs applications comme antithrombotiques.

La présente invention a pour objet, des héparosanes-N,O-sulfatés constitués par des chaînes ou par un mélange de chaînes de masse moléculaire entre 1500 et 15000 Da, ayant la structure disaccharidique répétée de formule I :

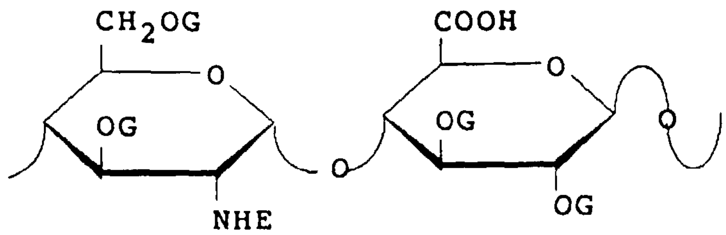

(I)

dans laquelle,

- E représente, dans 0 à 80 % des unités disaccharidiques desdits héparosanes-N,O-sulfatés, un groupe acétyle, et dans les unités disaccharidiques restantes, un groupe sulfate et éventuellement un atome d'hydrogène,
- G représente un atome d'hydrogène et un groupe sulfate,

et les sels pharmaceutiquement acceptables desdits héparosanes-N,O-sulfatés.

Le degré de sulfatation des héparosanes-N,O-sulfatés exprimé comme rapport sulfate/carboxyle, est de 1,5 à 3,0.

L'invention concerne aussi une composition d'héparosane-N,O-sulfaté contenant au moins 70 % en masse d'un héparosane-N,O-sulfaté décrit ci-dessus et objet de la présente invention, de préférence contenant au moins 90 % en masse d'un héparosane -N,O-sulfaté objet de la présente invention.

Les héparosanes-N,O-sulfatés objet de la présente invention peuvent être constitués de chaînes polysaccharidiques identiques de masse moléculaire bien définie, cette masse moléculaire étant située dans l'intervalle 1500-15000 Da. Ils peuvent aussi être constitués par un mélange de chaînes de masses moléculaires variables, ces masses moléculaires étant comprises entre 1500 et 15000 Da. La dispersion des masses moléculaires de ces chaînes peut-être plus ou moins importante. En effet, les héparosanes-N,O-sulfatés, objet de la présente invention peuvent être constitués par des chaînes ayant entres elles une différence de masse moléculaire d'au maximum environ 13500 Da, ou au contraire, seulement par des chaînes ayant entre elles une différence de poids moléculaire d'environ 300 Da, ce qui correspond à une unité de structure uronique (acide D-glucuronique ou ses dérivés) ou de structure glucosamine. Il est aussi évident qu'en fonction de la constitution de chaque héparosane-N,O-sulfaté, la masse moléculaire des chaînes ayant, soit la masse moléculaire la plus faible, soit la masse moléculaire la plus haute, peut correspondre à n'importe quelle valeur comprise entre 1500 et 15000 Da.

L'expression "G représente un atome d'hydrogène et un groupe sulfate" utilisée ci-dessus indique que G dans l'unité disaccharidique, représente pour certaines positions un atome d'hydrogène et dans les autres restantes, un groupe sulfate. De la même manière, E représente dans certaines unités disaccharidiques un groupe acétyle et dans le reste de ces unités, un groupe sulfate ou éventuellement un atome d'hydrogène. Les unités disaccharidiques des héparosanes-N,O-sulfatés, ne sont donc pas toutes identiques.

La formule I représente une structure répétitive disaccharidique formée par une unité glucosamine et une unité acide D-glucuronique. Lesdites unités peuvent être inversées, plus particulièrement, si l'on considère que la structure disaccharidique de formule I est répétée n fois, que l'unité non-réductrice des chaînes peut-être indifféremment soit une unité glucosamine, telle que représentée dans la formule I avec un groupe hydroxy en position 4, cette unité de glucosamine étant sulfatée ou non, soit un acide D-glucuronique contenant éventuellement une double liaison en position C4-C5 et étant sulfaté ou non. L'unité réductrice peut-être indifféremment soit un acide D-glucuronique, tel que représenté dans la formule I, substitué avec un hydrogène sur l'oxygène anomérique, soit une glucosamine, soit une structure 2,5-anhydromanno, telle qu'obtenue à la suite d'une dépolymérisation nitreuse (2,5-anhydro-D-mannose) suivie éventuellement d'une oxydation (acide 2,5-anhydro-D-mannonique) ou d'une réduction (2,5-anhydro-D-mannitol).

Les produits préférés sont ceux dont les deux extrémités, réductrice et non-réductrice des chaînes des héparosanes-N,O-sulfatés objet de la présente invention, sont des unités uroniques sulfatées ou non, de la glucosamine sulfatée ou non, de la N-acétyl glucosamine, sulfatée ou non, ou des unités ayant une structure 2,5-anhydromanno.

On préfère les héparosanes-N,O-sulfatés constitués de chaînes dont les deux extrémités, réductrice et non-réductrice, sont des unités uroniques sulfatées ou non, de la glucosamine sulfatée ou non, et de la N-acétyl glucosamine, sulfatée ou non.

La présente invention a également pour objet un procédé de préparation d'une composition contenant 70 % à 100 % d'un héparosane-N,O-sulfaté, objet de la présente invention, caractérisé en ce qu'il comprend la séquence des étapes suivantes :

- <u>étape a</u> : culture d'une souche d'<u>Escherichia coli</u> (K5), pour former un N-acétylhéparosane,

- étape b: isolement et purification du N-acétylhéparosane formé pour obtenir une composition contenant 70 % à 100 % d'un N-acétylhéparosane constitué par des chaînes ou par un mélange de chaînes de masse moléculaire entre 1500 et 15000 Da, ayant la structure disaccharidique répétée de formule II :

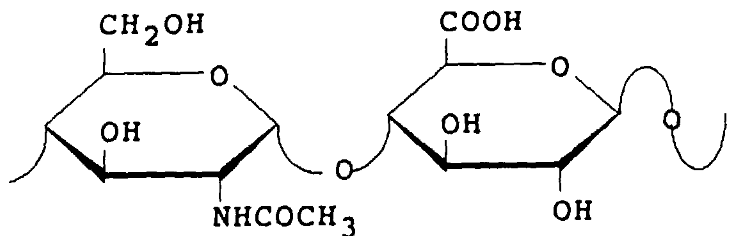

(II)

- étape c : désacétylation partielle de cette composition de N-acétylhéparosane pour obtenir une composition contenant 70 % à 100 % d'un héparosane constitué par des chaînes ou par un mélange de chaînes de masse moléculaire entre 1500 et 15000 Da, ayant la structure disaccharidique répétée de formule III :

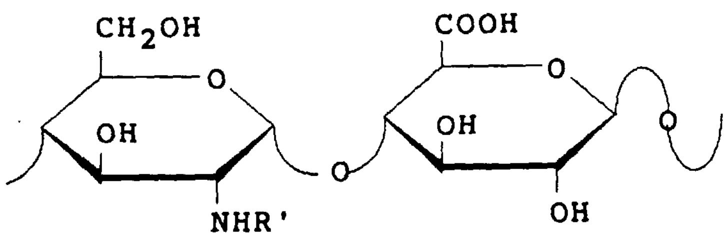

(III)

dans laquelle R' représente, dans 0 à 80 % des unités disaccharidiques, un groupe acétyle, et dans les unités disaccharidiques restantes un atome d'hydrogène,

- étape d :

  - soit N,O-sulfatation partielle de cette composition d'héparosane,
  - soit N,O-sulfatation partielle de cette composition d'héparosane suivie d'une étape de N-sulfatation totale,
  - soit une N-sulfatation totale ou partielle suivie d'une étape de O-sulfatation totale ou partielle,

et comporte éventuellement une ou plusieurs étapes de fractionnement des masses moléculaires effectuées à la fin des étapes a, b, c ou d.

Pour la préparation d'une composition contenant 70 % à 100 % d'un héparosane-N,O-sulfaté, objet de la présente invention et selon le procédé de l'invention, on utilise de manière préférentielle comme souche d'Escherichia coli (K5), la souche Escherichia coli SEBR 3282. Cette souche est une souche dérivée de la souche Bi 8337-41 (O10:K5:H4) ATCC 23506 (décrite notamment par D.S. Gupta et al FEMS Microbiology Letters, (1982), 14, 75-78 et W. Vann Eur. J. Biochem., (1981), 116, 359-364).

La souche Escherichia coli SEBR 3282 répond positivement au test de typage par le phage K5 spécifique selon la méthode de B. Kaiser et al (J. Clin. Microbiol., (1984), 19, 2, 264-266). Il s'agit donc bien d'une souche Escherichia coli (K5). Cette souche a été déposée auprès de la CNCM de l'Institut Pasteur, Paris, France, sous le N ° I-1013. On peut aussi utiliser un mutant, soit spontané soit induit de cette souche, comme aussi d'autres souches d'Escherichia coli (K5) appropriées par exemple la souche Bi 626-42 (012: K5:NM) ATCC 23508.

Le milieu de culture utilisé est de préférence un milieu riche en matière azotée, par exemple un milieu riche en extrait de levure et en hydrolysat de caséine, moyen peu coûteux, d'apport d'une quantité importante d'acides aminés.

La culture de la souche d'Escherichia coli (K5) est continuée de préférence au moins deux heures après l'arrêt de la croissance de la biomasse.

L'isolement et la purification du N-acétylhéparosane, pour obtenir une composition contenant 70 % à 100 % d'un N-acétylhéparosane constitué par un mélange de chaînes de masse moléculaire entre 1500 et 15000 Da, caractérisées par une structure disaccharidique répétée de formule II, sont réalisés par un procédé comprenant au moins une étape de précipitation et une étape de chromatographie d'échange d'ions. Cette étape est réalisée en utilisant de préférence une colonne Q-Sepharose ou une colonne équivalente. La précipitation est effectuée par un solvant organique approprié, et notamment un alcool, de préférence l'éthanol. Lors de ce procédé, le N-acétylhéparosane peut-être sous forme de sel, de préférence sous forme de sel de sodium.

A titre d'exemple, on peut schématiser le procédé préféré d'isolement et de purification comme suit :

- étape $a_1$ : Précipitation par l'éthanol,
- étape $b_1$ : Dialyse,
- étape $c_1$ : Précipitation par l'éthanol, puis déshydratation et séchage,
- étape $d_1$ : Purification par chromatographie d'échange d'anions,
- étape $e_1$ : Précipitation par l'éthanol de l'éluat, obtenu par l'étape $d_1$, déshydratation, séchage et broyage.

Concernant les étapes $a_1$, $b_1$, $c_1$, l'ordre avec lequel elles sont effectuées importe peu. Une des étapes $a_1$ ou $c_1$ peut être supprimée.

A l'étape $e_1$, la précipitation par l'éthanol n'est pas indispensable. Il est possible d'isoler le N-acétylhéparosane par d'autres méthodes comme par exemple évaporation sous vide de l'éluat, obtenu par l'étape $d_1$.

L'isolement et la purification du N-acétylhéparosane, pour obtenir une composition contenant 70 % à 100 % d'un N-acétylhéparosane constitué par un mélange de chaînes de masse moléculaire comprise entre 1500 et 15000 Da peut aussi être effectuée de la manière suivante :

- étape $a'_1$ : Dialyse,
- étape $b'_1$ : Purification en milieu acide, élimination des impuretés insolubles dans des solutions aqueuses de pH 3,5 et pH 1,8,
- étape $c'_1$ : Précipitation par l'éthanol, puis déshydratation et séchage,
- étape $d'_1$ : Hydrolyse alcaline et dialyse,
- étape $e'_1$ : Purification par chromatographie d'échange d'anions,
- étape $f'_1$ : Purification par chromatographie d'exclusion.

Ce procédé d'isolement et de purification est aussi un procédé préféré de l'invention.

L'hydrolyse alcaline est effectuée avec une solution de NaOH, à une température comprise entre 30-80°C.

En appliquant les procédés d'isolement et de purification, on peut utiliser comme produit de départ, soit la suspension obtenue à la fin de la culture et dans ce cas une filtration préalable est nécessaire, soit un produit qui est déjà soumis à une purification préliminaire effectuée selon un procédé qui comporte les étapes suivantes :

- étape $a''_1$ : Centrifugation de la suspension obtenue à la fin de la culture,
- étape $b''_1$ : Mise en contact du surnageant avec une solution alcaline,
- étape $c''_1$ : Préfiltration,
- étape $d''_1$ : Concentration sur membrane de seuil de coupure déterminé,
- étape $e''_1$ : Dialyse.

L'étape $e''_1$ n'est pas indispensable.

Comme solution alcaline, on peut utiliser une solution de NaOH 0,1 N.

De manière préférentielle, on procède à une purification préliminaire du produit obtenu à la fin de la culture, selon la méthode décrite ci-dessus.

La culture des souches d'Escherichia coli (K5) et les procédés d'isolement et de purifications ainsi que l'étape de purification préliminaire mentionnés ci-dessus, permettent d'obtenir des compositions de N-acétylhéparosanes contenant 70 % à 100 % d'un N-acétylhéparosane constitué par un mélange de chaînes de masse moléculaire entre 1500 et 15000 Da, ayant la structure disaccharidique répétée de formule II :

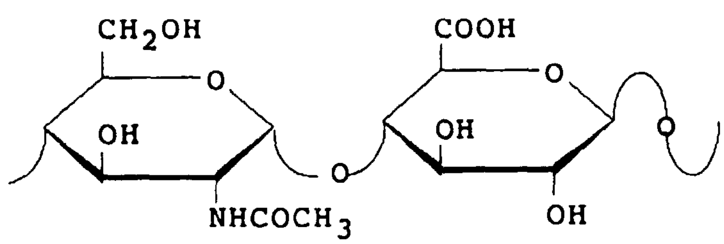

(II)

En effet, grâce aux procédés d'isolement et de purification décrits ci-dessus et à l'étape de la purification préliminaire, il est possible d'obtenir des compositions de N-acétylhéparosanes contenant au moins 80 % à 100 % d'un N-acétylhéparosane constitué par un mélange de chaînes de masse moléculaire entre 1500 et 15000 Da.

Les deux extrémités réductrice et non-réductrice des nouveaux N-acétylhéparosanes tels qu'obtenus selon le procédé décrit ci-dessus et en utilisant la souche d'Escherichia coli SEBR 3282 ou une autre souche appropriée comme indiqué précedemment, sont des unités uroniques ou de la N-acétylglucosamine.

Dans la majorité des chaînes, l'extrémité non-réductrice est une unité uronique de formule (a) :

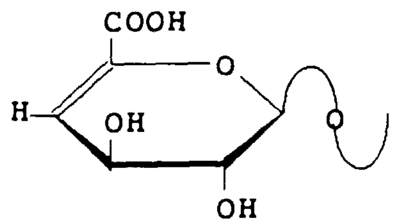

(a)

L'enrichissement éventuel de la composition obtenue en N-acétylhéparosane, constituée par un mélange de chaînes de masse moléculaire entre 1500 et 15000 Da, ayant une structure disaccharidique répétée de formule II, peut-être effectué à des degrés divers et notamment jusqu'à l'isolement dudit N-acétylhéparosane. L'enrichissement est réalisé par la mise en oeuvre de techniques classiques de fractionnement des masses moléculaires telles que la chromatographie de perméation de gel et ultrafiltration (A.A. Horner, Biochem. J., (1989), 262, 953-958; G. Pyler et al, J. Biol. Chem. (1988), 263, 11, 5197-5201 et U. Lindahl et al, J. Biol. Chem., (1984), 259, 20, 12368-12376). On peut aussi utiliser la méthode de fractionnement éthanolique (Demande de Brevet EP-A-0 287 477). Cette dernière méthode de fractionnement est particulièrement appréciée parmi d'autres méthodes envisageables.

Les N-acétylhéparosanes décrits ci-dessus sont utilisés comme intermédiaires pour la préparation des héparosanes-N,O-sulfatés objet de la présente invention mais aussi pour la préparation d'autres dérivés.

Pour préparer les héparosanes-N,O-sulfatés, objet de la présente invention, on peut aussi utiliser d'autres N-acétylhéparosanes connus, comme par exemple les N-acétylhéparosanes constitués principalement de chaînes polysaccharidiques ayant toutes le même nombre d'unités disaccharidiques, telles que décrites dans la demande de brevet EP-A-0 333 243, et notamment constituées de 6, 8, ou 10 unités "sucres", des fractions de N-acétylhéparosanes constituées de chaînes contenant en moyenne 10 unités monosaccharidiques (Gupta et al, FEMS Microbiology Letters (1983), 16, 13-17) ou de N-acétylhéparosanes de haute masse moléculaire qui peuvent être ensuite dépolymérisés selon les méthodes utilisées pour préparer les héparines de bas poids moléculaire.

En effet, les héparosanes-N,O-sulfatés objet de la présente invention peuvent être préparés par d'autres procédés à partir du polysaccharide (K5) connu, ayant une haute masse moléculaire. Dans ce cas, une étape de dépolymérisation est nécessaire.

Cette dépolymérisation peut-être effectuée avant la désacétylation du N-acétylhéparosane de haute masse moléculaire, après la désacétylation, après la N-sulfatation ou encore, après la N,O-sulfatation.

Comme il a été mentionné précedemment, la dépolymérisation peut-être éffectuée selon les méthodes décrites dans la littérature pour préparer les héparines de bas poids moléculaire, par exemple, par dépolymérisation au périodate, par des radicaux libres, par bétâ-élimination ou par action de l'acide nitreux (Demandes de Brevets ou Brevets EP-0 040 144, EP-0 037 319, EP-0 121 067). Ces méthodes sont données à titre d'exemples et ne sont pas limitatives. On peut utiliser toute autre méthode de dépolymérisation de glycosaminoglycanes.

Lorsque les héparosanes-N,O-sulfatés sont préparés par dépolymérisation, à partir d'un N-acétylhéparosane de haute masse moléculaire (préalablement désacétylé et éventuellement N-sulfaté) en le soumettant à l'action de l'acide nitreux, l'unité réductrice des héparosanes-N,O-sulfatés objet de la présente invention, peut avoir une structure 2,5-anhydromanno, et notamment de 2,5-anhydro-D-mannose. La dépolymérisation par action de l'acide nitreux peut être effectuée après l'étape de la N-désacétylation ou de la N-sulfatation. Quand cette étape est suivie d'une oxydation ou d'une réduction, on obtient des héparosanes-N,O-sulfatés ayant à l'unité reductrice, une structure respectivement acide 2,5-anhydro-D-mannonique ou 2,5-anhydro-D-mannitol.

La désacétylation partielle des compositions contenant 70 % à 100 % de N-acétylhéparosane, qui conduit à l'obtention des compositions contenant 70 % à 100 % et même 80 % à 100 % d'un héparosane constitué par un mélange de chaînes de masse moléculaire entre 1500 et 15000 Da, caractérisées par une structure disaccharidique répétée de formule III, indiquée ci-dessus, est réalisée par un traitement avec un agent désacétylant.

Comme agents désacétylants on peut mentionner le pentasulfure de phosphore, le fluoroborate de triéthyloxonium, la soude ou l'hydrazine, ces deux derniers agents étant particulièrement appréciés. On peut aussi utiliser les acides minéraux forts, tels que l'acide chlorhydrique, l'acide sulfurique etc.... La durée de la réaction dépend des conditions

opératoires choisies et notamment de la température et de la concentration de l'agent désacétylant dans le milieu réactionnel.

L'enrichissement de la composition de l'héparosane en héparosane constitué par des chaînes ou par un mélange de chaînes de masse moléculaire comprise entre 1500 et 15000 Da, caractérisées par une structure disaccharidique répétée de formule III, est réalisé par la mise en oeuvre de techniques classiques de fractionnement des masses moléculaires mentionnées ci-dessus (chromatographie de perméation gel, ultrafiltration et fractionnement éthanolique). Dans ce cas, on obtient des compositions contenant 90 % à 100 % en masse d'un héparosane constitué par un mélange de masse moléculaire entre 1500 et 15000 Da, ayant la structure disaccharidique répétée de formule III.

Pour la préparation d'héparosanes constitués d'un mélange de chaînes de masses moléculaires comprises entre 1500 et 15000 Da, il est aussi possible d'utiliser des N-acétylhéparosanes de haut poids moléculaire. Les héparosanes de haut poids moléculaire obtenus après désacétylation peuvent être dépolymérisés par les méthodes déjà décrites pour la préparation d'héparines de bas poids moléculaire et déjà mentionnées dans cette demande. Les produits de dépolymérisation peuvent être ensuite soumis à un fractionnement afin d'obtenir les héparosanes préférés de l'invention.

Les héparosanes constitués par un mélange de chaînes de masse moléculaire entre 1500 et 15000 Da, ayant la structure disaccharidique répétée de formule III :

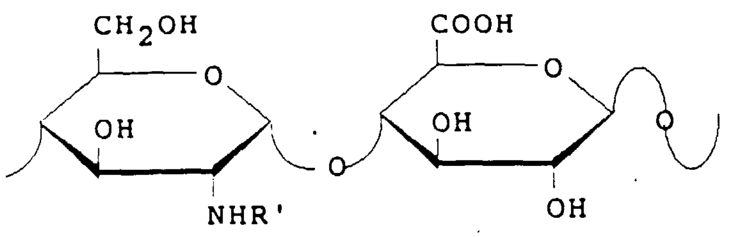

(III)

dans laquelle R' représente, dans 0 à 80 % des unités disaccharidiques, un groupe acétyle, et dans les unités disaccharidiques restantes un atome d'hydrogène, sont des produits nouveaux et à ce titre font aussi partie de l'invention.

Les héparosanes préférés de l'invention sont les héparosanes constitués par un mélange de chaînes de masse moléculaire entre 1500 et 15000 Da, caractérisées par une structure disaccharidique répétée de formule III dans laquelle le groupe acétyle (R') est présent à un taux inférieur ou égal à 60 %.

Les compositions d'héparosane qui contiennent au moins 70 % en masse d'un héparosane tel que décrit ci-dessus, font aussi partie de la présente invention.

Ces héparosanes et les compositions d'héparosane sont des intermédiaires utiles pour la préparation des héparosanes-N,O-sulfatés objet de la présente invention mais ils peuvent aussi être utilisés pour la préparation d'autres produits, par exemple des produits qui subissent une épimérisation, par la suite, au niveau de l'unité acide D-glucuronique.

Avant l'étape de la N,O-sulfatation partielle, les héparosanes peuvent être transformés en un sel de base organique ou en un sel d'ammonium quaternaire. Pour la formation du sel d'ammonium quaternaire des héparosanes, on utilise de manière préférentielle le tétrabutylammonium.

L'étape de la N,O-sulfatation partielle réalisée selon le procédé décrit dans le brevet français N ° 2 584 728 du 10.11.87 (correspondant à la demande FR-85.10787), est effectuée dans un solvant aprotique polaire, tel que le diméthylformamide, le diméthylsulfoxyde, l'hexaméthylphosphoramide ou l'acétonitrile ou un mélange de ces solvants, à l'aide par exemple d'un complexe d'anhydride sulfurique (trioxyde de soufre : $SO_3$) avec une base organique, telle que la triméthylamine, la triéthylamine ou la pyridine. Elle peut aussi être effectuée par l'acide chlorosulfonique en solution dans la pyridine. On utilise de préférence le complexe trioxyde de soufre-pyridine.

Il est aussi possible d'utiliser d'autres agents de sulfatation, notamment ceux qui sont rapportés par E.E. Gilbert dans Chemical Review, (1962), 62, 549-589. La réaction de N,O-sulfatation est en général effectuée à une température comprise entre 0° et 100°C, de préférence entre 10° et 50°C, pendant une durée comprise entre 6 h et 14 h.

Lors du procédé de la préparation, à la fin de la réaction de N,O-sulfatation partielle, la composition de l'héparosane-N,O-sulfaté contenant 70 % à 100 % d'un héparosane-N,0-sulfaté objet de la présente invention, est précipitée par addition de chlorure de sodium jusqu'à obtention d'une solution de 0,33 M en chlorure de sodium, puis d'une quantité adéquate d'éthanol. Le précipité formé est repris dans une solution de chlorure de sodium 0,5 M. Cette solution est ensuite neutralisée. Après addition d'une quantité adéquate d'éthanol, le précipité formé est isolé, repris avec de l'eau ultra-purifiée, dialysé contre celle-ci, lyophilisé et séché.

L'étape de la N,O-sulfatation, ainsi que les étapes de purification de la composition de l'héparosane-N,O-sulfaté décrites dans le paragraphe précédent, peuvent être répétées une ou plusieurs fois. Le processus de la purification

est donné à titre d'exemple et n'exclut pas des procédés équivalents.

Cette étape de N,O-sulfatation est de préférence suivie d'une étape de N-sulfatation totale, réalisée en général dans un solvant aqueux, avantageusement de pH basique, avec un agent de sulfatation, tel qu'un complexe d'anhydride sulfurique avec une base organique, par exemple la triméthylamine.

A la fin de l'étape de N-sulfatation totale, la composition de l'héparosane-N,O-sulfaté est précipitée après addition de chlorure de sodium, jusqu'à l'obtention d'une solution de 0,5 M en chlorure de sodium, et d'éthanol. Le précipité formé est ensuite remis en solution dans une solution de chlorure de sodium 0,5 M, reprécipité par l'éthanol, puis repris dans l'eau ultra-purifiée, dialysé contre celle-ci, lyophilisé et séché.

L'enrichissement de la composition de l'héparosane-N,O-sulfaté en héparosane-N,O-sulfaté, est réalisé par la mise en oeuvre des techniques classiques de fractionnement des masses moléculaires, déjà mentionnées. Il est intéressant d'effectuer cet enrichissement. .

Les héparosanes-N,O-sulfatés, objet de la présente invention et les compositions d'héparosane-N,O-sulfaté contenant au moins 70 % d'un nouvel héparosane-N,O-sulfaté, objet de la présente invention, sont avantageusement obtenus par un procédé comportant une réaction finale de N-sulfatation. E représente alors pour les structures répétées dans la formule (I), le groupe acétyle et le groupe sulfate.

Il est aussi possible d'obtenir les héparosanes-N,O-sulfatés, objet de la présente invention, en procédant d'abord à une N-sulfatation suivie d'une O-sulfatation. Cette variante de procédé est utilisée de manière préférentielle.

La N-sulfatation est réalisée à l'aide d'un complexe de trioxyde de soufre avec une base organique telle que la triméthylamine, la triéthylamine ou la pyridine. Elle peut aussi être effectuée par l'acide chlorosulfonique en solution dans la pyridine. On utilise avantageusement le complexe trioxyde de soufre avec la triméthylamine, et la réaction de la N-sulfatation est réalisée à une température comprise entre 20-80°C en milieu aqueux alcalin. A la fin de la réaction de N-sulfatation, le produit ainsi obtenu est précipité par addition d'une quantité adéquate d'éthanol. Le précipité formé est repris avec de l'eau ultra-purifiée, dialysé contre celle-ci, lyophilisé et séché. Le processus de la purification est donné à titre d'exemple et n'exclut pas des procédés équivalents. Les étapes de purification peuvent être répétées plusieurs fois.

Selon le procédé de l'invention, avant l'étape de la O-sulfatation, l'héparosane N-sulfaté est transformé de préférence en un sel de base organique ou en un sel d'ammonium quaternaire. Pour la formation du sel d'ammonium quaternaire on utilise de manière préférentielle, le tetrabutylammonium.

La réaction de O-sulfatation est réalisée dans le formamide ou un autre solvant, chimiquement équivalent, à l'aide par exemple d'un complexe de trioxyde de soufre avec une base organique telle que la triméthylamine, la triéthylamine ou la pyridine. On utilise de préférence un complexe trioxyde de soufre-pyridine. La réaction de O-sulfatation est en général effectuée à une température comprise entre 10°C et 50°C.

L'héparosane-N,O-sulfaté est ensuite précipité par addition dans le milieu réactionnel de chlorure de sodium jusqu'à obtention d'une solution de 0,33 M en NaCl, puis, d'une quantité adéquate d'éthanol, comme dans le cas de la N,O-sulfatation. On procède ensuite à une purification de la composition de l'héparosane-N,O-sulfaté. Les différentes étapes ont déjà été décrites de manière détaillée, ci-dessus (précipitation par l'éthanol, dialyse etc...).

Selon l'invention, l'héparosane-N,O-sulfaté préféré comporte au moins 90 % en masse de chaînes de masse moléculaire inférieure à 11000 Da. On apprécie que cet héparosane-N,O-sulfaté contienne moins de 0,2 μmole/mg, de groupe amino ($NH_2$).

Le groupe acétyle est de préférence présent à un taux inférieur ou égal à 60 % et on apprécie que le degré de sulfatation, exprimé comme le rapport sulfate/carboxyle soit compris entre 1,5 et 3,0.

Les produits préférés de l'invention sont les héparosanes-N,O-sulfatés constitués de chaînes ayant une masse moléculaire moyenne d'environ 4000 à 7000 Da et un degré de sulfatation, exprimé comme le rapport sulfate/carboxyle, compris entre 1,7 et 3, comme aussi les héparosanes-N,O-sulfatés, N-désacétylés à environ 80 % (taux du groupe acétyle présent environ 20 %) et étant constitués par au moins 70 % en masse de chaînes de masses moléculaires comprises entre 5000 et 7000 Da et ayant un degré de sulfatation de 1,8 à 2,5, ou encore les héparosanes-N,O-sulfatés étant constitués par au moins 70 % en masse de chaînes de masses moléculaires comprises entre 10000-12000 Da, N-désacétylés à environ 80 % (taux du groupe acétyle présent environ 20 %) et ayant un degré de sulfatation de 1,8 à 2,5, ou encore les héparosanes-N,O-sulfatés, N-désacétylés à environ 40 % (taux du groupe acétyle présent environ 60 %) et étant constitués par au moins 70 % en masse de chaînes de masses moléculaires comprises entre 6000 et 8000 Da et ayant un degré de sulfatation de 2,0 à 2,8.

Les héparosanes-N,O-sulfatés préférés de l'invention sont plus spécialement :

- Les héparosanes-N,O-sulfatés, N-désacétylés à environ 80 % (taux du groupe acétyle présent environ 20 %) étant constitués par au moins 80 % en masse de chaînes de masses moléculaires comprises entre 2300 Da et 7200 Da et ayant un degré de sulfatation de 1,8 à 2,5,

- Les héparosanes-N,O-sulfatés, N-désacétylés à environ 80 % (taux du groupe acétyle présent environ 20 %) étant

constitués par au moins 80 % en masse de chaînes de masses moléculaires comprises entre 3300 Da et 7700 Da et ayant un degré de sulfatation de 1,8 à 2,5,

- Les héparosanes-N,O-sulfatés, N-désacétylés à environ 80 % (taux du groupe acétyle présent environ 20 %) étant constitués par au moins 70 % en masse de chaînes de masses moléculaires comprises entre 6900 Da et 13500 Da et ayant un degré de sulfatation de 1,8 à 2,5,

- Les héparosanes-N,O-sulfatés, N-désacétylés à environ 40 % (taux du groupe acétyle présent environ 60 %) étant constitués par au moins 80 % en masse de chaînes de masses moléculaires comprises entre 4000 Da et 10300 Da et ayant un degré de sulfatation de 2,0 à 2,8.

Comme sels des héparosanes-N,O-sulfatés on entend tous les sels pharmaceutiquement acceptables. Ces sels sont obtenus par des méthodes classiques décrites notamment pour la préparation des sels de l'héparine (Brevet US 4.168.377).

Le procédé d'obtention des héparosanes-N,O-sulfatés décrit ci-dessus ainsi que les méthodes de purifications permettent l'obtention des héparosanes-N,O-sulfatés sous forme de sel de sodium. A partir de ces sels, on peut obtenir en appliquant les méthodes utilisées pour préparer les différents sels d'héparine ou l'héparine non salifiée ("L'héparine, fabrication, structure, propriétés, analyses", J.P. Duclos, (1984) pp 81-83, Masson Ed.-France) soit d'autres sels d'héparosanes-N,O-sulfatés, soit des héparosanes-N,O-sulfatés non salifiés.

Les héparosanes-N,O-sulfatés, objet de la présente invention possèdent des propriétés pharmacologiques et biochimiques intéressantes et tout à fait surprenantes par rapport aux enseignements de l'art antérieur.

Notamment, contrairement aux produits sulfatés de l'antigène K5 décrits dans le brevet EP-A-0 333 243, qui ont une activité antiangiogénique et antitumorale, avec un rapport favorable de ces activités par rapport aux propriétés anticoagulantes, les héparosanes-N,O-sulfatés de la présente invention possèdent une bonne activité régulatrice de la coagulation. Cette activité est très supérieure à celle du dermatane-sulfate sur les différents paramètres de la coagulation, et elle peut plutôt être comparée à celle de l'héparine.

Plus particulièrement, l'activité anti-IIa, ATIII ou HCII dépendante, de produits représentatifs de l'invention a été déterminée selon les méthodes décrites par D. Dupouy et al dans Thrombosis and Haemostasis, (1988), 60, 2, 236-239, pour le cofacteur II de l'héparine (HCII) et par M.L. Larsen et al dans Thrombosis Research, (1978), 13, 2, 285-288 pour l'antithrombine (ATIII).

Dans les deux cas, le test consiste à mesurer in vitro l'effet inhibiteur du produit étudié sur la thrombine purifiée (facteur IIa) en présence d'HCII ou ATIII purifiés dans le dosage de l'activité amidolytique de la thrombine, vis-à-vis d'un substrat chromogène. Comme il possède l'activité anti IIa HCII dépendante la plus forte, le dermatane-sulfate, préparé selon la méthode décrite par H.W. Stuhlsatz et al dans "The Methodology of Connective Tissue Research" (1976) 137-146, il est utilisé comme produit de référence dans le test de mesure de cette activité, le résultat étant exprimé en mg de dermatane-sulfate (DS) équivalents en activité à 1 mg du produit étudié (mg DS équiv/mg).

L'activité anti-Xa (titre Yin-Wessler) desdits produits représentatifs de l'invention a été mesurée par la méthode décrite par E.T. Yin et al dans J. Lab. Clin. Med. (1973), 81, 2, 298-310, tandis-que leur activité anticoagulante globale a été mesurée selon le test APTT décrit par R.R. Proctor et al dans Am. J. Clin. Path. (1961), 36, 212-219.

Tous les produits testés ont montré une activité anti-IIa HCII dépendante nettement supérieure à celle du dermatane-sulfate. L'activité anti-IIa ATIII dépendante et le titre de Yin-Wessler, bien qu'inférieurs à ceux des héparines, se sont montrés supérieurs à ceux du dermatane-sulfate. Leur titre en APTT est d'environ 2 à environ 20 fois supérieur à celui du dermatane-sulfate, et peut arriver jusqu'à 60 % de celle de l'héparine.

Les héparosanes-N,O-sulfatés de l'invention présentent donc une spécificité d'action et une activité anticoagulante particulièrement intéressantes. La faible masse moléculaire de ces produits leur donne par ailleurs des propriétés pharmacocinétiques aussi très intéressantes.

Les héparosanes-N,O-sulfatés de la présente invention sont très peu toxiques ; leur toxicité est parfaitement compatible avec leur utilisation comme médicaments.

L'invention s'étend donc aussi aux compositions pharmaceutiques renfermant comme principe actif un héparosane-N,O-sulfaté, objet de la présente invention, un de ses sels, ou une composition d'héparosane N,O-sulfaté contenant au moins 70 % de cet héparosane-N,O-sulfaté ou l'un de ses sels, en association avec un ou plusieurs véhicules pharmaceutiques appropriés.

Ces compositions pharmaceutiques sont utiles notamment pour le traitement, à titre préventif ou curatif, des troubles de la paroi vasculaire tels que l'athérosclérose et l'artériosclérose et des états d'hypercoagulabilité observés par exemple à la suite d'opérations chirurgicales, de développements tumoraux ou de dérèglements de la coagulation induits par des activateurs bactériens, viraux ou enzymatiques.

La posologie peut largement varier en fonction de l'âge, du poids et de l'état de santé du patient, de la nature et de la sévérité de l'affection ainsi que de la voie d'administration.

Cette posologie comprend l'administration d'une ou plusieurs doses d'environ 1 mg à 1 g par jour, de préférence d'environ 5 mg à 500 mg par jour par exemple de l'ordre de 200 mg par jour par voie intraveineuse, ou par voie sous-cutanée, en administrations discontinues ou à intervalles réguliers, ou d'une dose journalière de l'ordre de 200 mg à 1000 mg par jour par voie orale.

Ces doses peuvent être naturellement ajustées pour chaque patient en fonction des résultats observés et des analyses de sang effectuées auparavant.

L'invention est illustrée par les exemples ci-après.

## N-ACETYLHEPAROSANES

### EXEMPLE 1

### Préparation d'un N-acétylhéparosane majoritairement de faible masse moléculaire (Procédé I)

**1) Culture de la souche bactérienne Escherichia coli (K5) et séparation d'un filtrat contenant du N-acétylhéparosane**

On ensemence 400 ml du milieu B, de composition précisée dans le tableau I ci-après, avec la souche Escherichia coli SEBR 3282 (déposée auprès de la CNCM de l'Institut Pasteur, Paris France, sous le N° I-1013) et on incube sous agitation pendant 2 h à 37°C.

La préculture obtenue est alors transférée dans un fermenteur de 18,5 l contenant 11 l du milieu A, de composition précisée dans le tableau I ci-après et on incube pendant 4 h à 37°C et pH égal à 7,4, la pression partielle en oxygène étant maintenue à 5332 Pa (40 mm Hg) par régulation de l'injection d'air (jusqu'à 20 l/min) et de l'agitation. On ajoute alors du glucose en introduisant de façon continue une solution stérile contenant 600 g/l de glucose à raison de 250 ml/h pendant 8 h.

On poursuit la culture dans les mêmes conditions de température, de pH et de pression partielle en oxygène pendant 10 h après l'arrêt de l'addition de glucose. Le suivi de la DO ($\lambda = 600$ nm) du milieu de culture permet d'affirmer qu'il n'y a pas de croissance de la biomasse pendant les 12 dernières heures de la culture.

On refroidit alors le bouillon de culture à 25°C puis on le filtre à travers une membrane de porosité 0,22 µm. On obtient ainsi environ 12 l de filtrat contenant du N-acétylhéparosane.

**TABLEAU I**

**Composition et préparation du milieu A et du milieu B**

**MILIEU A**

Le milieu A est préparé par la réunion des trois solutions stériles ci-dessous :

Solution N°1

dans 700 ml d'eau ultra-purifiée dissoudre dans l'ordre :

| | |
|---|---|
| Complexant: N'[Tris-(hydroxyméthyl) méthyl] glycine (Tricine commercialisé par Fluka®) | 360 mg |
| $FeSO_4$, $7H_2O$ | 280 mg |
| $CaCl_2$, $2H_2O$ | 6,7 mg |
| $MgCl_2$, $6H_2O$ | 1270 mg |
| $K_2SO_4$ | 500 mg |
| KCl | 5000 mg |
| Hydrolysat de caséine (source principale d'acides aminés) HY CASE SF (commercialisé par Sheffield®) | 25000 mg |
| Extrait de levure (commercialisé par Difco®) | 18000 mg |
| Solution d'oligo-éléments (cf tableau II ci-après) | 1 ml |

Agent antimousse Struktol J673 (commercialisé par Schill et Seilache®) : quelques gouttes à la pipette Pasteur.

Ajuster le pH à 7,4 avec une solution de KOH (d = 1,38) et compléter à 850 ml avec de l'eau ultra-purifiée. Autoclaver

le milieu 45 min à 120°C,

Solution N°2

Dans environ 40 ml d'eau ultra-purifiée, dissoudre 5 g de $K_2HPO_4$ puis ajuster à 50 ml avec le même solvant. Filtrer la solution obtenue à travers un filtre de porosité 0,2 µm.

Solution N°3

Dissoudre 20,7 g de glucose dans une quantité adéquate d'eau ultra-purifiée et ajuster le volume à 100 ml avec le même solvant. Autoclaver à 110°C pendant 30 minutes.

**MILIEU B**

La préparation du milieu B est identique à celle du milieu A, à la différence près, qu'il convient d'ajouter en plus 20 g de tampon pH = 7,2 : (acide 3-morpholinopropanesulfonique) après l'addition de l'agent anti-mousse.

TABLEAU II

| **Préparation de la solution d'oligo-éléments utilisée dans la préparation du milieu A et du milieu B** | |
|---|---|
| dans 800 ml d'eau ultra-purifiée dissoudre (dans l'ordre) : | |
| $H_3BO_3$ | 500 mg |
| $Na_2MoO_4,2H_2O$ | 1930 mg |
| $CoCl_2,6H_2O$ | 11850 mg |
| $CuSO_4,5H_2O$ | 25 mg |
| $ZnSO_4,7H_2O$ | 2000 mg |
| $AlCl_3,6H_2O$ | 2410 mg |

Ajouter 100 ml d'acide chlorhydrique de densité 1,19 et compléter à 1000 ml avec de l'eau ultra-purifiée.

**2) Isolement et purification d'un N-acétylhéparosane majoritairement de faible masse moléculaire :**

Etape a - Précipitation éthanolique:

On ajoute au filtrat environ 48 l d'éthanol à 95 % (v/v) et on laisse le mélange précipiter et décanter à 4°C pendant 8 h. On élimine le surnageant par pompage, puis centrifugation, et on reprend le culot de centrifugation dans environ 1 l d'eau ultra-purifiée.

Etape b - Dialyse:

On dialyse pendant 24 h la solution obtenue à l'étape précédente placée dans un boudin NOJAX 40 portant une membrane à base de cellulose et de porosité 24Å, contre de l'eau ultra-purifiée (1 volume de solution/ 6 volumes d'eau, renouvelés après 2 h, 8 h et 16 h). Cette opération permet d'éliminer les petites molécules présentes dans le milieu de culture, tels que sels, sucres, acides aminés, oligonucléotides et oligopeptides.

Etape c - Précipitation, déshydratation et séchage :

On ajoute à 1 volume. de la solution dialysée, 0,5 M de NaCl et 4 volumes d'éthanol. On laisse se former le précipité pendant 5 min à température ambiante. On centrifuge à 5000 g pendant 20 min. On reprend les culots de centrifugation dans l'éthanol, on agite la suspension obtenue, et on la laisse reposer pendant h à température ambiante. On répète les opérations de centrifugation et de mise en suspension. On centrifuge à nouveau à 5000 g pendant 20 min. On sèche les culots de centrifugation obtenus dans une étuve sous vide à 40°C pendant 24 h.

Etape d - Broyage en poudre :

Les culots de centrifugation secs sont broyés à l'aide d'un mortier dans des conditions anhydres.

Etape e - Chromatographie d'échange d'anions :

On reprend les culots de centrifugation broyés, dans un tampon appelé tampon D, de composition Tris-HCl 20 mM pH 7,5, à raison de 100 ml/g. La solution obtenue est chromatographiée sur une colonne d'échange d'anions forts comportant une matrice d'agarose réticulée avec des groupes ammonium quaternaires (le "Q-Sepharose fast flow" de Pharmacia®) préalablement équilibré avec le tampon D, à raison de 50 ml de gel pour 1 g de poudre. On lave le gel avec une quantité suffisante de tampon D pour le retour à la ligne de base de la détection UV à 214 nm, puis avec une solution de pipérazine 25 mM dont le pH a été ajusté à 3,5. On élue avec une solution de pH 3,5 ayant une composition : NaCl 0,5 M et pipérazine 25 mM. L'éluat est neutralisé à l'aide d'une solution de NaOH 5 N.

Etape f - Précipitation, déshydratation, séchage et broyage :

On répète les opérations décrites dans les étapes c et d ci-dessus, sans ajout de chlorure de sodium. Le N-acétylhéparosane obtenu à l'issue de l'étape f, est appelé lot A.

Une variante du procédé de purification consiste à effectuer successivement les étapes a, c, b, d, e, et f. Le N-acétylhéparosane ainsi obtenu est appelé lot B.

**3) Caractérisation du N-acétylhéparosane obtenu à l'issue des différentes étapes de purification :**

Spectre de résonance magnétique nucléaire (RMN)

Les spectres RMN du proton et du carbone $^{13}C$ sont comparés à ceux du N-acétylhéparosane décrit par W.E. Vann (Eur. J. Biochem., (1981), 116, 59-364).

L'étude des spectres obtenus avec le N-acétylhéparosane du lot A et du lot B, confirme l'identité chimique du produit au N-acétylhéparosane décrit par W.F. Vann. Il s'agit de chaînes de polymères constituées de structures répétées de β-D-glucuronyl-1,4-α-N-acétyl-D-glucosaminyl-(1,4).

Détermination de la répartition des masses moléculaires par chromatographie d'exclusion

La répartition des masses moléculaires est déterminée par HPLC d'exclusion dans les conditions suivantes :

* Colonne constituée de billes de silice de diamètre 10 μm et de porosité 250Å.
* Eluant: solution aqueuse de sulfate de sodium 0,5 M.
* Débit : 1 ml/min
* Détection UV à λ = 205 nm.
* L'étalonnage est effectué à l'aide d'une gamme d'oligosaccharides dérivés de l'héparine, de masses moléculaires suivantes : 1324, 1883, 2436, 3411, 3996, 4535, 4995, 5365, 6150, 6671, 7542, 8655, 10088, 11561, 12950, 14809, 17387 et 22674 Da.

Compte tenu de cette gamme d'étalons, seules les masses moléculaires entre 934 Da et 56703 Da sont prises en compte. On admet que la densité optique détectée est proportionnelle à la quantité de N-acétylhéparosane. Toutefois, la précision de la méthode diminue de manière exponentielle pour les hautes masses moléculaires et notamment supérieures à 20000 Da.

Le profil d'élution de la chromatographie d'exclusion du lot A est représenté sur la figure 1. On constate à l'examen de la figure 1, que la distribution est polydispersée et qu'elle comporte un pic majoritaire à environ 4700 Da. Une fraction pondérale au moins égale à 70 % du lot A possède une masse comprise entre 1700 et 8000 Da.

Un chromatogramme très semblable est obtenu lors de l'analyse du lot B. Le pic majoritaire de la distribution est à environ 5000 Da. Une fraction pondérale au moins égale à 70 % du lot B, possède une masse moléculaire comprise entre 1500 et 8000 Da.

Suivi de la répartition des masses moléculaires par électrophorèse sur gel de polyacrylamide

On a soumis ces échantillons à analyser ainsi qu'un marqueur de fin de migration comprenant du bleu de bromophénol, à une électrophorèse en tampon Tris-borate dans un gel de polyacrylamide à 15 % obtenu par polymérisation d'un mélange 29/1 d'acrylamide et de N,N'-méthylène-bis-acrylamide. La migration est effectuée sous 40 mA pendant environ 4 h sur un gel de 18 cm de longueur jusqu'à la sortie du marqueur de fin de migration. Le gel est alors coloré au bleu alcian, puis à l'argent, selon la technique de S. Pelkonen et al (J. Bact., (1983), 170, 6, 2646) spécifique aux polysaccharides acides.

Cette analyse par électrophorèse a été effectuée sur le produit partiellement purifié obtenu à l'issue de l'étape a et le produit purifié, lot A ou lot B, issu de l'étape finale, dans le but de vérifier l'absence de modification importante de la répartition des masses moléculaires du N-acétylhéparosane au cours de la purification.

L'observation des profils obtenus pour le produit partiellement purifié obtenu à l'issue de l'étape a et le produit purifié à l'étape finale (lot A ou lot B), ne met pas en évidence de différences importantes (présence de bandes d'intensité comparable aux mêmes distances de migration). Il n'y a donc pas de modification importante de la répartition des masses moléculaires du N-acétylhéparosane au cours de sa purification.

Dosage des acides uroniques

La quantité d'acide uronique par unité de masse du produit purifié (lot A ou lot B), obtenu à l'issue de l'étape finale, à été déterminée par colorimétrie selon la méthode décrite par T. Bitter, (Analytical Biochemistry, (1962), 4, 330-334). Cette méthode de dosage est basée sur la réaction des glycosaminoglycanes avec le carbazole en milieu acide à chaud, qui provoque une coloration en rose proportionnelle à la quantité d'acide uronique libérée.

Pour le lot A, le produit partiellement purifié obtenu à l'issue de l'étape d et le produit purifié obtenu à l'issue de l'étape finale f ont respectivement un taux d'acide uronique de 1,3 et 2,1 μmol/mg.

Pour le lot B le produit purifié à l'issue de l'étape finale a également un taux d'acide uronique de 2,1 μmol/mg.

Spectrophotométrie dans le domaine ultra-violet et visible

Le produit purifié (lot A), est dissous dans de l'eau ultra-purifiée et la solution obtenue (C = 1 mg/ml) est placée dans une cuve de 1 cm de trajet optique. Son spectre d'absorption est enregistré entre 200 et 600 nm.

Le spectre obtenu permet d'affirmer, en particulier sur la base de l'absorption à 256 nm, que le lot A contient moins de 1 % d'ADN.

Dosage des protéines totales

Le kit "protein assay" commercialisé par BIORAD est utilisé pour doser les protéines totales. La méthode de dosage est basée sur le fait que la longueur d'onde du maximum d'absorbance d'une solution acide de bleu brillant de Coomassie g-250 passe de 465 nm à 595 nm, lorsque des protéines viennent s'y fixer (Reisner et al., Anal Biochem, (1975), 64, 509).

Le taux de protéines totales du lot A est inférieur à 1,5 %.

Dosage des groupes amino libres ($NH_2$)

Ce dosage a été effectué selon la méthode décrite par Zensaku Yosizawa et al., dans Biochemica et Biophysica Acta, (1967), 141, 358-365.

Le paramètre taux de $NH_2$ (exprimé en μmol/mg) est un indicateur des quantités d'unités β-D-glucuronyml-1,4-α-N-acétyl-D-glucosaminyl-(1,4) désacétylées et des contaminants qui contiennent un groupement amino libre.

Le lot A et le lot B ont chacun un taux de $NH_2$ 0,05 μmol/mg. Le rapport $NH_2$/acide glucuronique = 0,05/2,1 est inférieur à 2,5 %. Il y a donc (en mol) pour 100 unités β-D-glucuronyl-1,4-α-N-acétyl-glucosaminyl-(1,4) moins de 2,5 unités disaccharidiques de ce type désacétylées.

**EXEMPLE 2**

**Préparation d'un N-acétylhéparosane majoritairement de faible masse molécuaire (Procédé II)**

**1) Culture de la souche bactérienne Escherichia coli (K5) et séparation d'un filtrat contenant du N-acétylhéparosane**

La culture de la souche Escherichia coli SEBR 3282 et la séparation d'un filtrat contenant du N-acétylhéparosane ont été réalisées selon la méthode décrite dans l'Exemple 1.

**2) Isolement et purification d'un N-acétylhéparosane majoritairement de faible masse moléculaire**

Etape a - Dialyse

375 ml de filtrat sont soumis à une dialyse selon le procédé décrit dans l'Exemple 1, [2) Isolement et purification

d'un N-acétylhéparosane majoritairement de faible masse moléculaire, Etape b]. Après dialyse on obtient environ 1020 ml de solution purifiée.

Etape b - Purification en milieu acide

On ajoute à la solution dialysée une quantité adéquate d'une solution d'HCl 5 N pour obtenir un pH égal à 3,5. On élimine par centrifugation le précipité formé, puis on acidifie la solution avec le même acide (HCl 5N) pour obtenir un pH égal à 1,8. Un précipité peut se former, qui sera éliminé par centrifugation. On neutralise ensuite la solution à l'aide d'une solution de NaOH 5 N.

Etape c - Précipitation, déshydratation et séchage

On ajoute à la solution-neutralisée une quantité adéquate de chlorure de sodium pour avoir une solution de 0,5 M en NaCl, puis on ajoute 4 volumes d'éthanol. On laisse se former le précipité pendant 5 mn à température ambiante. On centrifuge à 5000 g pendant 20 min. On reprend les culots de centrifugation dans l'éthanol, on agite la suspension obtenue, et on la laisse reposer pendant 1 h à température ambiante. On répète les opérations de centrifugation et de mise en suspension. On centrifuge à nouveau à 5000 g pendant 20 min. On sèche les culots de centrifugation obtenus dans une étuve sous vide à 40°C pendant 24 h.

Etape d - Hydrolyse alcaline et dialyse

Le produit obtenu à d'étape précédente après séchage est mis en solution à 2,5 % (p/v) dans une solution de NaOH 0,25 N. On maintient la solution ainsi obtenue 2 heures à 50 °C. On neutralise ensuite à l'aide d'une solution d'HCl 5 N et on soumet ensuite la solution contenant le polysaccharide à une dialyse selon le procédé décrit dans l'Exemple 1, [ 2) Isolement et purification d'un N-acétylhéparosane majoritairement de faible masse moléculaire, Etape b].

Après dialyse, on obtient environ 990 ml de solution.

Etape e - Chromatographie d'échange d'anions

A la solution dialysée on ajoute des quantités adéquates de pipérazine, d'EDTA et de triton X-100 (Prolabo®) pour avoir respectivement des concentrations de 25 mM en pipérazine, 2 mM en EDTA et 0,2 % (v/v) en triton X-100. Le pH est ensuite ajusté à 3,5 à l'aide d'une solution d'HCl 5 N. On dépose cette solution sur une colonne Q-Sepharose Fast Flow de 400 ml, équilibrée dans un tampon pipérazine contenant 25 mM de pipérazine, 2 mM d'EDTA et 0,2 mM de triton X-100 (pH = 3,5). On lave par le tampon pipérazine, on élue avec une solution de NaCl 0,5 M, puis on précipite par 4 volumes d'éthanol. On sèche sous vide à 40°C. On obtient ainsi environ 9,85 g de N-acétylhéparosane.

Etape f - Chromatographie d'exclusion

4 g du produit obtenu au stade précédent sont mis en solution dans 60 ml d'une solution tampon de composition tris-HCl 20 mM pH 7,5 et NaCl 1 M, puis passés sur une colonne de de 200 ml d'octyl Sepharose® préalablement équilibrée avec le même tampon. A la fraction non retenue, on ajoute 4 volumes d'éthanol. On lave le précipité formé et on le sèche à 40°C sous vide.

On obtient ainsi 3,90 g de N-acétylhéparosane.

**3) Caractérisation du N-acétylhéparosane obtenu à l'issue des différentes étapes de purification**

Spectre de résonance magnétique nucléaire RMN

L'étude des spectres RMN du proton et du carbone $^{13}C$ obtenus avec cet N-acétylhéparosane, confirme l'identité chimique du produit au N-acétyl-héparosane décrit par W.F. Vann (Eur. J. Biochem. (1981) 116, 59-364).

Détermination de la répartition des masses moléculaires par chromatographie d'exclusion

La répartition des masses moléculaires est déterminée par HPLC d'exclusion selon la méthode utilisée pour la détermination de la répartition des masses moléculaires de N-acétylhéparosanes décrits dans l'Exemple 1. Une fraction pondérale au moins égale à 86 % des chaînes qui constituent le lot issu de l'Exemple 2 possède une masse moléculaire comprise entre 1500 et 15000 Da.

Dosage des acides uroniques

Le N-acétylhéparosane issu de l'étape e, a un taux d'acide uronique de 1,94 µmole/mg.

Spectrophotométrie dans le domaine de l'ultra-violet et visible

Le spectre obtenu permet d'affirmer que le N-acétylhéparosane obtenu contient moins de 1 % d'ADN.

Dosage des protéines totales

Le taux de protéines totales de ce lot de N-acétylhéparosane est inférieur à 1 %.

Dosage des groupes amino libres ($NH_2$)

Le taux de $NH_2$ est inférieur à 0,1 µmole/mg

**EXEMPLE 3**

**Préparation d'un N-acétylhéparosane majoritairement de faible masse moléculaire (Procédé III)**

**1) Culture de la souche Escherichia coli (K5)**

La culture de la souche Escherichia coli SEBR3282 a été réalisée selon la méthode décrite dans l'Exemple 1. On obtient environ 12 l de culture contenant du N-acétylhéparosane.

**2) Purification préliminaire**

Etape a - Centrifugation

A la fin de la culture, on centrifuge la suspension obtenue (12 l) à 8000 t/min (soit entre 11000 et 14000 g) pendant 20 min.

Etape b - Mise en contact avec une solution alcaline

Après la centrifugation, le culot est éliminé et le surnageant est mis en contact avec une solution de NaOH 0,1 N pendant environ 1 heure.

Etape c - Préfiltration

La solution obtenue à l'étape précédente est soumise à une préfiltration sur filtre 3M® serie 300 en polypropylène.

Etape d - Concentration sur membrane de seuil de coupure déterminé.

Le filtrat obtenu à l'étape c est concentré sur cartouche à fibres creuses Amicon®, seuil de coupure 10000 Da ou équivalent. On obtient ainsi une solution enrichie en N-acétylhéparosane de faible masse moléculaire.

Etape e - Dialyse

On dialyse la solution enrichie en N-acétylhéparosane de faible masse moléculaire contre de l'eau ultra-purifiée toujours sur système Amicon®, à un facteur de dilution très élevé (> 10000).

**3) Isolement et purification d'un N-acétylhéparosane majoritairement de faible masse moléculaire:**

On procéde comme il est indiqué dans l'Exemple 1 [ 2) Isolement et purification d'un N-acétylhéparosane majoritairement de faible masse moléculaire, Etape c - Etape f] et on obtient un N-acétylhéparosane ayant des caractéristiques similaires à celles du lot A, ou comme il est indiqué dans l'Exemple 2 [ 2) Isolement et purification d'un N-acétylhéparosane majoritairement de faible masse moléculaire, Etape a - Etape f].

**HEPAROSANES-N,O-SULFATES**

**EXEMPLE 4**

**Modifications chimiques du N-acétylhéparosane majoritairement de faible masse moléculaire issu de l'Exemple 1**

**1) Modifications chimiques du lot B**

Etape a - Désacétylation partielle :

On dissout 500 mg du lot B dans 10 ml de solution de NaOH 1N. On porte la solution à 50°C et on laisse réagir à cette température pendant 8 h sous agitation. On neutralise alors la solution avec une solution d'HCl 2N, on la dialyse contre de l'eau ultra-purifiée puis on la lyophilise.

Etape b - Formation du sel de tétrabutylammonium:

Le lyophilisat précédent est repris dans 20 ml d'eau ultra-purifiée. La solution obtenue est envoyée sur une colonne d'échange d'ions à base de polystyrèneréticulé par le divinylbenzène (Dowex 50 W 8 de Dow Chemical®), préalablement conditionnée en milieu acide, de façon à régénérer la forme acide du produit. La solution est alors mélangée avec 0,4 ml d'une solution à 40 % de tétrabutylammonium. On lyophilise.

Etape c - N,O-sulfatation partielle :

On dissout 421 mg du sel obtenu à l'étape précédente dans 35 ml de diméthylformamide et on ajoute 3,71 g de complexe trioxyde de soufre-pyridine (commercialisé par Aldrich® sous la réf. S755-6). On laisse réagir sous agitation pendant 6 h à température ambiante. A un volume du milieu réactionnel on ajoute du chlorure de sodium jusqu'à l'obtention d'une solution de concentration 0,33 M en chlorure de sodium, puis 2 volumes d'éthanol. On laisse se former le précipité. On centrifuge et on élimine le surnageant. Le culot de centrifugation est repris dans une solution de NaCl 0,5 M, qu'on neutralise. On ajoute ensuite 2 volumes d'éthanol. On laisse se former le précipité, on centrifuge et on reprend le culot de centrifugation avec de l'eau ultra-purifiée. On dialyse contre de l'eau ultra-purifiée et on lyophilise.

On répète l'ensemble des opérations décrites dans le paragraphe ci-dessus.

Le lyophilisat obtenu présente les caractéristiques suivantes :

* taux d'acide uronique: 1,11 µmol/mg
* taux de $NH_2$ libre: 0,01 µmol/mg
* taux de sulfatation : 2,64 par unité disaccharidique

Les taux d'acide uronique et de $NH_2$ sont mesurés comme décrit dans l'Exemple 1.

Le taux de sulfatation, également appelé rapport sulfate/carboxyle, est le nombre moyen de groupes sulfate par groupe carboxyle ; il est mesuré par la méthode conductimétrique de dosage du groupe sulfate et du groupe carboxyle décrite par B. Casu et al., dans Carbohydrate Research, (1975), 39, 168-176.

**2) Modifications chimiques du lot A**

Le lot A est divisé en trois fractions aliquotes, appelées lot A1, lot A2 et lot A3.

Etape a - Désacétylation partielle et gel filtration :

Les lots A1, A2 et A3 ont été traités comme décrit à l'étape a, pour le lot B ci-dessus, à la différence près que seul le lot A1 est dialysé et lyophilisé. Les lots A1, A2 et A3 sont ensuite fractionnés par gel filtration (également appelé chromatographie de perméation gel ou chromatographie d'exclusion), dans les conditions suivantes :

Support de billes de diamètres 25-75 µm à base d'allyldextrane réticulé au N,N'-méthylène-bis-acrylamide (Sephacryl S 300 HR commercialisé par Pharmacia®). Eluant utilisé : solution de NaCl 0,5 M.

On réalise les mélanges des fractions correspondant à un Kav de 0,46 à 1 pour l'héparosane issu du lot A1, de 0,43 à 1 pour l'héparosane issu du lot A2 et de 0,43 à 0,64 pour l'héparosane issu du lot A3. Ces fractions sont appelées par la suite "héparosanes gel filtrés".

Le Kav est un coefficient habituellement utilisé en chromatographie d'exclusion, et permet de reproduire le fractionnement par chromatographie d'exclusion. Il est défini par la formule :

$$kav = \frac{Ve-Vt}{Vo-Vt}$$

dans laquelle

Ve = volume d'élution de la fraction considérée
Vo = volume d'exclusion
Vt = volume total du gel.

La répartition des masses moléculaires de l'héparosane issu du lot A1 immédiatement après désacétylation partielle ainsi que des héparosanes gel filtrés issus des lots A1, A2 et A3, est déterminée par chromatographie d'exclusion selon la technique décrite dans l'Exemple 1.

Les profils d'élution obtenus avant et après gel filtration pour l'héparosane issu du lot A1 sont représentés respectivement sur les figures 2 et 3. Certains résultats déduits des profils d'élution sont rassemblés dans le tableau III ci-après, dans lequel PMo représente la masse moléculaire telle qu'une fraction pondérale de 1 % du produit ait une masse moléculaire supérieure à PMo, $PM_1$ représente le masse moléculaire telle qu'une fraction pondérale de 10 % du produit ait une masse moléculaire supérieure à $PM_1$, $PM_3$ la masse moléculaire telle qu'une fraction pondérale de 10 % du produit ait une masse moléculaire inférieure à $PM_3$, $PM_2$ représente la masse moléculaire correspondant au maximum d'absorption.

## TABLEAU III

### Répartition des masses moléculaires de l'héparosane, issu du lot A1 (non gel filtré) et des héparosanes gel filtrés issus des lots A1, A2 et A3

| | PMo | $PM_1$ | $PM_2$ | $PM_3$ |
|---|---|---|---|---|
| **Héparosane non gel filtré issu du lot A1** | 41400 | 9600 | 4400 | 1400 |
| **Héparosane gel filtré issu du lot A1** | 10700 | 6900 | 4400 | 1600 |
| **Héparosane gel filtré issu du lot A2** | 12700 | 7000 | 4500 | 1500 |
| **Héparosane gel filtré issu du lot A3** | 10300 | 6900 | 4500 | 1500 |

On constate en comparant les figures 2 et 3, que la gel filtration permet d'éliminer la fraction minoritaire de haute masse moléculaire de l'héparosane.

Ce résultat apparaît clairement dans le tableau III où l'on constate une forte diminution de PMo à la suite de l'opération de gel filtration. L'héparosane de chacun des lots contient au moins 90 % en masse de chaînes de masses moléculaires inférieures à 7000 Da.

Les héparosanes gel filtrés issus des lots A1 et A2 sont ensuite traités comme décrit dans l'Exemple 1 [ 2) Isolement et purification d'un N-acétylhéparosane majoritairement de faible masse moléculaire, Etape c].

L'héparosane gel filtré issu du lot A3 n'est pas précipité mais est dialysé contre de l'eau ultra-purifiée.

Les taux d'acide uronique et des groupes amino libres sont mesurés comme décrit dans l'Exemple 1.

Les résultats obtenus sont rassemblés dans le tableau IV ci-après. Le taux d'acétyle résiduel a été évalué en considérant qu'il y a autant de groupes glucuronyle que de groupes glucosaminyle.

Le taux de désacétylation a été calculé comme étant égal au rapport du taux de groupes amino libres sur le taux

d'acide uronique.

Spectre de résonance magnétique nucléaire

L'étude du spectre de résonance magnétique nucléaire du proton obtenu par l'héparosane gel filtré issu du lot A3, a permis de conclure que le produit possède la structure attendue.

Le taux de désacétylation calculé par intégration à été trouvé égal à 44 %. Cette valeur est voisine de celle calculée en utilisant le rapport du taux de groupes amino libres sur le taux d'acide uronique (41 %).

## TABLEAU IV

### Caractéristiques des héparosanes gel filtrés issus des lots A1, A2 et A3

| | Taux d'acide uronique dosé (µmol/mg) | Taux de $NH_2$ dosé (µmol/mg) | Taux d'acétyle résiduel calculé (µmol/mg) | Taux de désacétylation |
|---|---|---|---|---|
| Héparosane gel filtré issu du lot A1 | 2,3 | 1,10 | 1,20 | 48 % |
| Héparosane gel filtré issu du lot A2 | 2,4 | 1,00 | 1,40 | 42 % |
| Héparosane gel filtré issu du lot A3 | 2,6 | 1,05 | 1,55 | 41 % |

Etape b - N,O-sulfatation partielle :

On traite les héparosanes gel filtrés issus des lots A1, A2 et A3 désacétylés de la façon décrite précédemment pour le lot B (Etape c N,O-sulfatation partielle), à la différence près que l'on ne répète pas les opérations décrites dans le premier paragraphe.

Pour l'héparosane issu du lot A3, après la première précipitation, le culot est repris dans l'eau ultra-purifiée, dialysé contre de l'eau ultra-purifiée et laissé en solution.

Les caractéristiques des produits N,O-sulfatés sont rassemblées dans le tableau V ci-après :

## TABLEAU V

**Caractéristiques des héparosanes-N,O-sulfatés issus des lots A1, A2 et A3 à l'issue de la réaction de N,O-sulfatation partielle**

| | Taux de $NH_2$ (µmol/mg) | Taux de sulfatation (rapport sulfate/carboxyle) |
|---|---|---|
| Héparosane-N,O-sulfaté issu du lot A1 | 0,10 | 1,9 |
| Héparosane-N,O-sulfaté issu du lot A2 | 0,10 | 1,9 |
| Héparosane-N,O-sulfaté issu du lot A3 | 0,10 | 1,8 |

On remarque qu'après la réaction de N,O-sulfatation le taux de $NH_2$ résiduel (0,10 µmol/mg) est supérieur à celui des N-acétylhéparosanes purifiés (0,05 µmol/mg). La sulfatation n'est donc pas complète sur l'atome d'azote.

Etape c - N-sulfatation totale

On mélange dans un volume de 20 ml d'eau ultra-purifiée par gramme de produit N,O-sulfaté engagé, 1 partie pondérale de produit N,O-sulfaté, 1 partie pondérale de bicarbonate de sodium, 1 partie pondérale du complexe trioxyde de soufre-triméthylamine et on laisse réagir à 55°C sous agitation pendant 20 h. On dilue ensuite le mélange réactionnel (facteur de dilution 10), puis on ajuste la conductivité de la solution obtenue à celle d'une solution de chlorure de sodium 0,5 M. Ensuite sont réalisées une précipitation par ajout de 2 volumes d'éthanol, une centrifugation suivie d'une reprise des culots de centrifugation par une solution de NaCl 0,5 M, puis une seconde précipitation par ajout de 2 volumes d'éthanol. Après reprise dans de l'eau ultra-purifiée et dialyse contre de l'eau ultra-purifiée, les produits sont lyophilisés et séchés à 40°C sous vide.

Spectre de résonance magnétique nucléaire

L'étude du spectre RMN du $^{13}C$ de l'héparosane-N,O-sulfaté issu du lot A3 montre que pour ce composé, l'alcool en position 6 du groupe glucosaminyle est entièrement sous forme d'ester sulfurique.

Certaines caractéristiques des héparosanes N,O-sulfatés obtenus, déterminées selon les méthodes décrites précédemment sont rassemblées dans le tableau VI ci-après :

## TABLEAU VI

### Caractéristiques des produits obtenus à l'issue de la N-sulfatation totale pour les lots A1, A2 et A3

| | Taux d'acide uronique (µmol/mg) | Taux de $NH_2$ (µmol/mg) | Taux de désacétylation | Taux de sulfatation (rapport sulfate /carboxyle) |
|---|---|---|---|---|
| Héparosane-N,O-sulfaté issu du lot A1 | 1,20 | 0,02 | 48 % | 2,2 |
| Héparosane-N,O-sulfaté issu du lot A2 | 1,30 | 0,02 | 42 % | 2,2 |
| Héparosane-N,O-sulfaté issu du lot A3 | 1,35 | 0,02 | 41 % | 2,1 |

On constate que le taux de $NH_2$ résiduel (0,02 µmol/mg) est faible et inférieur à celui du N-acétylhéparosane purifié (0,05 µmol/mg pour le lot A et le lot B) ainsi qu'à celui de l'héparosane-N,O-sulfaté obtenu à l'issue de la N,O-sulfatation partielle (précisé dans le tableau V). Ceci démontre le caractère total de la réaction de la N-sulfatation.

La répartition des poids moléculaires des produits obtenus après N-sulfatation est analysée par la technique décrite dans l'Exemple 1.

Le profil d'élution obtenu pour le produit issu du lot A1 est représenté sur la figure 4. Des profils d'élution très semblables sont obtenus pour les produits issus des lots A2 et A3.

Certains résultats déduits des profils d'élution sont rassemblés dans le tableau VII. La définition de PMo, $PM_1$, $PM_2$ et $PM_3$ est identique à celle indiquée pour le tableau III.

## TABLEAU VII

### Répartition des masses moléculaires des produits obtenus à l'issue de la N-sulfatation totale pour les lots A1, A2 et A3.

| | PMo | $PM_1$ | $PM_2$ | $PM_3$ |
|---|---|---|---|---|
| **Lot A1** | 14700 | 9000 | 5700 | 2600 |
| **Lot A2** | 17500 | 10000 | 5800 | 3400 |
| **Lot A3** | 11600 | 7600 | 5000 | 1800 |

L'héparosane-N,O-sulfaté de chacun des lots contient au moins 90 % en masse de chaînes de masses moléculaires inférieures à 10000 Da.

En effet, les lots A1, A2 et A3, contiennent 80 % de chaînes de masses moléculaires comprises respectivement entre 2600 Da et 9000 Da, 3400 et 10000 Da et 1800 et 7600 Da.

**EXEMPLE 5**

**Modifications chimiques du N-acétylhéparosane majoritairement de faible masse moléculaire issu de l'Exemple 2. Préparation des héparosanesN,O-sulfatés, dérivés N-désacétylés à 80 %**

Le N-acétylhéparosane utilisé a été préparé selon le procédé décrit dans l'Exemple 2. Le taux d'acide uronique de ce produit est de 2,12 µmoles/mg. La détermination de la répartition des masses moléculaires a été effectuée par chromatographie d'exclusion, selon la méthode décrite à l'Exemple 1. Une fraction pondérale au moins égale à 87,5 % possède des chaînes de masses moléculaires comprises entre 1500 et 15000 Da. Le pic majoritaire de la distribution est à environ 4900 Da. Ce lot de N-acétylhéparosane est appelé lot C.

Etape a - Désacétylation partielle

On dissout 2,5 g du lot C dans 50 ml d'une solution de NaOH 2 N. On porte la solution à 50°C et on laisse réagir à cette température pendant 8 h sous agitation. On ajuste le pH à 8 à l'aide d'une solution de HCl 2 N, on la dialyse contre de l'eau ultra-purifiée, puis on la lyophilise.

On obtient ainsi 1,96 g de produit.

Taux de N-désacétylation

Le dosage des groupes amino libres ($NH_2$) indique que le N-acétylhéparosane a été N-désacétylé à 80 %.

Etape b - N-sulfatation

Le lyophilisat précédent est repris dans 70 ml d'eau et on ajoute 2,5 g de $Na_2CO_3$ et de 2,5 g de complexe trioxyde de soufre-triméthylamine. On laisse réagir 20 heures à 55°C.

On amène la conductivité de la solution réactionnelle à celle d'une solution de NaCl 0,5 M par addition d'eau déminéralisée. On précipite par 4 volumes d'éthanol. On centrifuge. On dissout à nouveau le précipité dans une solution de NaCl 0,5 M. On précipite par 4 volumes d'éthanol. On centrifuge.

On reprend le culot de centrifugation avec de l'eau ultra-purifiée, on dialyse cette solution selon le procédé déjà décrit (Exemple 1) et on lyophilise.

On obtient environ 2 g de produit.

Etape c - Formation du sel de tétrabutylammonium

Le lyophilisat obtenu à l'étape précédente est transformé en sel de trétrabutylammonium selon le procédé décrit dans l'Exemple 4 [ 1) Modifications chimiques du lot B, Etape b].

On obtient 2,7 g de sel.

Etape d - O-sulfatation

On dissout 2,680 g du sel obtenu à l'étape précédente dans 196 ml de formamide et on ajoute 11,27 g de complexe trioxyde de soufre-pyridine. On laisse réagir sous agitation pendant 6 heures à 30°C. On ajoute 1 volume d'une solution de NaCl 2 M pour 5 volumes de solution réactionnelle et on ajuste le pH à 7 à l'aide d'une solution de NaOH. On re-précipite par 2 volumes d'éthanol, on centrifuge et on re-dissout le culot dans une solution de NaCl 0,5 M. On ajoute ensuite 2 volumes d'éthanol. On laisse se former le précipité, on centrifuge et on reprend le culot de centrifugation avec 80 ml d'eau ultra-purifiée. On ajoute 20 ml d'une solution de NaCl 2 M puis, 4 volumes d'éthanol. On laisse se former le précipité et on centrifuge.

Etape e - Gel filtration

Le produit obtenu à l'étape d est repris avec de l'eau ultra-purifiée puis fractionné par gel filtration selon les conditions décrites dans l'Exemple 4 [ 2) Modifications chimiques du lot A, Etape a]. On détermine les masses moléculaires des chaînes qui constituent la composition de l'héparosane-N,O-sulfaté par chromatographie d'exclusion, selon le procédé décrit dans l'Exemple 1. On regroupe d'une part les fractions contenant des chaînes ayant des masses moléculaires comprises entre 1500 et 12000 Da [Solution C(A)] et d'autre part, des fractions contenant des héparosanes-N,O-sulfatés composés de chaînes de masses moléculaires de 2000 à 30000 Da [(Solution C(M)].

A la solution C(A), on ajoute 4 volumes d'éthanol. On laisse se former le précipité ; on centrifuge et on reprend le culot de centrifugation avec de l'eau ultra-purifiée. On dialyse contre de l'eau ultra-purifiée et on lyophilise.

On obtient 1,8 g de produit.

L'héparosane-N,O-sulfaté ainsi obtenu est appelé lot C1.

Certaines caractéristiques de cet héparosane-N,O-sulfaté, déterminées selon les méthodes décrites précédemment sont rassemblées dans le tableau VIII ci-après.

## TABLEAU VIII

## Caractéristiques du produit correspondant au lot C1

| | Taux d'acide uronique (μmol/mg) | Taux de $NH_2$ (μmol/mg) | Taux de désacétylation | Taux de sulfatation (rapport sulfate /carboxyle) |
|---|---|---|---|---|
| **Lot C1** | 1,6 | 0,013 | 80 % | 1,9 |

La répartition du poids moléculaire du produit a été évaluée par la technique décrite dans l'Exemple 1. Certains résultats déduits du profil d'élution sont rassemblés dans le tableau IX.

## TABLEAU IX

### Répartition des masses moléculaires du produit correspondant au lot C1

| | PMo | $PM_1$ | $PM_2$ | $PM_3$ |
|---|---|---|---|---|
| Lot C1 | 9600 | 7200 | 5621 | 2367 |

La définition de PMo, $PM_1$, $PM_2$ et $PM_3$ est identique à celle indiquée dans le tableau III.

L'héparosane-N,O-sulfaté du lot C1 contient 95 % en masse de chaînes comprises entre 1500 et 15000 Da.

Spectre de résonance magnétique nucléaire (RMN)

Des spectres RMN du proton et du carbone $^{13}C$ sont obtenus avec l'héparosane-N,O-sulfaté du lot C1 (spectres réalisés sur AMX 500, solvant $D_2O$).

L'étude des spectres RMN du proton et du carbone $^{13}C$ confirme la structure attendue du produit. Il s'agit bien d'un héparosane-N,O-sulfaté.

L'étude du rapport d'intensité des protons des sucres sur les protons des acétyles du spectre du proton, conduit à proposer un taux de désacétylation de 84 %. Cette valeur est voisine de celle calculée en utilisant le rapport du taux de groupes amino libres sur le taux d'acide uronique (80 %).

Le spectre RMN du carbone $^{13}C$, confirme notamment que la glucosamine est N-sulfatée presque totalement. L'acide D-glucuronique n'est pas sulfaté en position 2 et 3.

Etape f - Gel filtration

A la solution C(M), on ajoute 4 volumes d'éthanol. On laisse se former le précipité, on centrifuge, on reprend le culot à l'eau ultra-purifiée, on dialyse et on lyophilise.

Le lyophilisat est ensuite dissout dans une solution de NaCl 0,5 M et fractionné par gel filtration selon les conditions définies à l'Etape e.

On regroupe d'une part des fractions contenant des chaînes ayant des masses moléculaires comprises entre 1300 et 21000 Da [Solution C(B)] et d'autre part, celles de14000 à 37000 Da [(Solution C(C)].

Ces deux solutions sont traitées comme indiqué précedemment pour la solution C(A) et on obtient après lyophilisation par la solution C(B), le lot C2 et par la solution C(C), le lot C3.

Le tableau X indique certains résultats déduits des profils d'élutions pour les produits du lot C2 et du lot C3.

## TABLEAU X

### Répartition des masses moléculaires des produits correspondant aux lots C2 et C3

| | PMo | $PM_1$ | $PM_2$ | $PM_3$ |
|---|---|---|---|---|
| Lot C2 | 14600 | 9600 | 7597 | 5950 |
| Lot C3 | 28656 | 17320 | 10512 | 7975 |

La définition de PMo, $PM_1$, $PM_2$ et $PM_3$ est identique à celle indiquée dans le tableau III.

L'héparosane-N,O-sulfaté correspondant au lot C2 contient environ 99 % en masse de chaînes dont la masse moléculaire est comprise entre 1500 et 15000 Da, et l'héparosane-N,O-sulfaté correspondant au lot C3 contient environ 73 % en masse, de chaînes dont la masse moléculaire est comprise entre 1500 et 15000 Da.

**EXEMPLE 6**

**Modifications chimiques du N-acétylhéparosane majoritairement de faible masse moléculaire issu de l'Exemple 2 - Préparation d'un héparosane-N,O-sulfaté ; dérivé N-désacétylé à 40 %, ayant un taux de sulfatation de 2,6**

Le N-acétylhéparosane utilisé comme matière première a été préparé selon le procédé décrit dans l'Exemple 2. Le taux d'acide uronique de ce produit est 1,96 μmoles/mg. Ce lot est appelé lot D.

Etape a - Désacétylation partielle

On dissout 3,7 g du lot D dans 74 ml de NaOH 1 N, et on traite selon le procédé décrit dans l'Exemple 5, (Etape a). La désacétylation est effectuée sous azote.

Après lyophilisation on obtient 2,91 g de produit.

Taux de N-désacétylation

Le dosage des groupes amino-libres ($NH_2$) du produit obtenu après lyophilisation indique que le N-acétylhéparosane a été N-désacétylé à 40 %.

Etape b - N-sulfatation

Le produit obtenu au stade précédent a été traité avec 3,7 g du complexe trioxyde de soufre-triméthylamine, en présence de 3,7 g de $Na_2CO_3$ et selon le procédé décrit dans l'Exemple 5 (Etape b).

On obtient ainsi environ 3 g d'un héparosane-N-sulfaté.

Etape c - Formation du sel de tétrabutylammonium

Environ 2 g de l'héparosane N-sulfaté obtenu au stade précédent sont transformés selon la méthode décrite dans l'Exemple 5 (Etape c), en sel de tétrabutylammonium.

On obtient 2,99 g de lyophilisat.

Etape d - O-sulfatation

On fait réagir 2,98 g du sel obtenu à l'étape précédente avec 14 g de complexe trioxyde de soufre-triméthylamine, selon le procédé décrit dans l'Exemple 5, (Etape d), puis on procède aux précipitations et purifications décrites dans ce même Exemple 5 (Etape d).

On obtient 2,8 g de produit.

Etape e - Gel filtration

Le produit obtenu à l'étape précédente est fractionné par gel filtration en utilisant la méthode et le matériel décrit dans l'Exemple 4, [ 2) Modifications chimiques du lot A, Etape a].

La répartition des masses moléculaires des fractions de l'héparosane-N,O-sulfaté est déterminée par chromatographie d'exclusion selon la technique décrite dans l'Exemple 1. On isole les fractions constituées des héparosanes-N,O-sulfatés dont la majorité des chaînes ont un poids moléculaire compris entre 1500 et 15000 Da.

Ces fractions sont ensuite concentrées, et soumises à une dialyse contre de l'eau ultra-purifiée. On réalise ensuite une précipitation par ajout de 5 volumes d'éthanol, on centrifuge, on dissout le précipité dans une solution de NaCl 0,5 M et on répète l'opération de la précipitation.

Le produit purifié ainsi obtenu est soumis à un deuxième fractionnement en utilisant le protocole mentionné au début de cette étape. La répartition des masses moléculaires des fractions est déterminée par chromatographie d'exclusion selon la méthode déjà décrite. On recueille les fractions contenant des produits ayant des masses moléculaires comprises entre 4000 et 8000 Da.

Les fractions sont soumises à une dialyse puis les produits sont précipités par addition d'éthanol. Le précipité est

récupéré et dissout dans une solution de NaCl 0,5 M. La solution ainsi obtenue est dialysée contre de l'eau ultra-purifiée puis lyophilisée.

On obtient ainsi environ 1 g d'un héparosane-N,O-sulfaté appelé lot D1.

Les caractéristiques et la répartition des masses moléculaires de cet héparosane-N,O-sulfaté sont indiquées respectivement dans les tableaux XI et XII.

## TABLEAU XI

### Caractéristiques du produit correspondant au lot D1

| | Taux d'acide uronique (μmol/mg) | Taux de $NH_2$ (μmol/mg) | Taux de désacétylation | Taux de sulfatation (rapport sulfate /carboxyle) |
|---|---|---|---|---|
| Lot D1 | 1,53 | 0,01 | 40 % | 2,60 |

## TABLEAU XII

### Répartition des masses moléculaires du produit correspondant au lot D1

| | PMo | $PM_1$ | $PM_2$ | $PM_3$ |
|---|---|---|---|---|
| Lot D1 | 15430 | 10305 | 6850 | 4047 |

La définition de PMo, $PM_1$, $PM_2$ et $PM_3$ est identique à celle indiquée dans le tableau III.

L'héparosane-N,O-sulfaté du lot D1 contient 80 % en masse de chaînes comprises entre 4047 et 10305 Da et environ 98,5 % en masse de ces chaînes ont une masse moléculaire comprise entre 1500 et 14600 Da. La figure 5 représente le profil d'élution obtenu pour cet héparosane-N,O-sulfaté.

Spectre de résonance magnétique nucléaire (RMN)

Des spectres RMN du proton et du carbone $^{13}C$ sont obtenus avec l'héparosane-N,O-sulfaté du lot D1 (spectres réalisés sur AMX 500, solvant $D_2O$).

L'étude des spectres RMN du proton et du carbone $^{13}C$ confirme la structure attendue du produit. Il s'agit bien d'un héparosane-N,O-sulfaté.

Le spectre RMN du carbone $^{13}C$ confirme notamment qu'aucun groupe amino du motif glucosamine n'est sous la forme amino libre ($NH_2$). La glucosamine est entièrement sulfaté en position C6, par contre, tous les groupes hydroxy de l'acide glucuronique ne sont pas sulfatés.

**EXEMPLE 7**

**Modifications chimiques d'un N-acétylhéparosane majoritairement de faible masse moléculaire issu de l'Exemple 2. Préparation d'un héparosane-N,O-sulfaté ; dérivé N-désacétylé à 40 %, ayant un taux de sulfatation de 3.**

Comme matière première on utilise un lot de N-acétylhéparosane préparé selon le procédé décrit dans l'Exemple 2. Ce lot est appelé lot E, et a un taux d'acide uronique de 2 μmoles/mg.

La répartition du poids moléculaire de ce N-acétylhéparosane a été évaluée par la technique décrite dans l'Exemple

1. Cet N-acétylhéparosane contient environ 75 % en masse de chaînes comprises entre 1500 et 15000 Da et la masse moyenne de ces chaînes est d'environ 10900 Da. La masse moléculaire de l'espèce majoritaire est de 5135 Da.

Etape a- Désacétylation partielle

Le N-acétylhéparosane a été N-désacétylé à 40 % selon le protocole mentionné dans l'Exemple 6 (Etape a). Pour effectuer cette N-désacétylation, on utilise 2,5 g de matière première que l'on dissout dans 50 ml de NaOH 1N. A la fin de la réaction, le pH du milieu réactionnel est ajusté à 6 à l'aide d'HCl puis concentré.

Taux de N-désacétylation

Le dosage des groupes amino libres ($NH_2$) indique que le N-acétylhéparosane a été N-désacétylé à 40 %.

Etape b- Gel-filtration

On utilise une colonne de Sephacryl S 300 HR (5 cm x 100 cm) équilibrée en NaCl 0,5 M.

La répartition des masses moléculaires de l'héparosane contenu dans les différentes fractions est déterminée par chromatographie d'exclusion. On regroupe les fractions contenant des chaînes ayant une masse moléculaire de 6000 à 20000 Da, on concentre au Rotavapor, on précipite par 4 volumes d'éthanol et on sèche le précipité formé.

On obtient ainsi environ 1 g de produit.

Etape c - Formation du sel de tétrabutylammonium et N,O-sulfatation partielle.

Pour former le sel de tétrabutylammonium, on utilise 300 mg du produit obtenu à l'étape précédente et on applique le procédé décrit dans l'Exemple 4 [ 1) Modifications chimiques du lot B, Etape b]. On obtient 490 mg de sel que l'on dissout dans 30 ml de formamide. On procède ensuite à une N,O-sulfatation partielle selon le procédé décrit dans l'Exemple 4 [1) Modifications chimiques du lot B, Etape c- premier paragraphe].

A partir de 490 mg de sel que l'on fait réagir avec 2,25 g de complexe trioxyde de soufre-pyridine, on obtient 406 mg d'héparosane-N,O-sulfaté.

Etape d- N-sulfatation totale et gel-filtration

372 mg du produit obtenu à l'étape ci-dessus sont dissous dans 15 ml d'une solution de $Na_2CO_3$ à 5 % et traités avec 372 mg de complexe trioxyde de soufre-triméthylamine, selon la méthode décrite dans l'Exemple 4 [ 2) Modifications chimiques du lot A, Etape c].

Le produit obtenu après N-sulfatation totale est ensuite fractionné par gel-filtration en utilisant une colonne Sephacryl S 300 HR (2,5 cm x 100 cm) et en appliquant le protocole déjà décrit.

Les fractions ayant une masse moléculaire de 6000 à 20000 Da, sont regroupées, concentrées au Rotavapor, dialysées extensivement contre de l'eau ultra-purifiée froide et lyophilisées.

On obtient ainsi 260 mg d'un héparosane-N,O-sulfaté appelé lot E1.

Les caractéristiques de ce produit sont rassemblées dans le tableau XIII.

Le tableau XIV indique la répartition des masses moléculaires de chaînes qui constituent le lot E1.

Cet héparosane-N,O-sulfaté contient environ 75 % en masse de chaînes de masses moléculaires entre 1500 et 15000 Da et environ 65 % en masse de chaînes ayant des masses moléculaires entre 7700 et 15000 Da.

## TABLEAU XIII

### Caractéristiques du produit correspondant au lot E1

| | Taux d'acide uronique ($\mu$mol/mg) | Taux de $NH_2$ ($\mu$mol/mg) | Taux de désacétylation | Taux de sulfatation (rapport sulfate /carboxyle) |
|---|---|---|---|---|
| Lot E1 | 1,35 | 0,01 | 40 % | 3 |

## TABLEAU XIV

### Répartition des masses moléculaires du produit correspondant au lot E1

| | PMo | $PM_1$ | $PM_2$ | $PM_3$ |
|---|---|---|---|---|
| Lot E1 | 31238 | 19671 | 12029 | 7748 |

La définition de PMo, $PM_1$, $PM_2$ et $PM_3$ est identique à celle indiquée dans le tableau III.

**EXEMPLE 8**

**Préparation de deux lots d'un héparosane-N,O-sulfaté ; dérivés N-désacétylés à 80 %, ayant un taux de sulfatation de 2,25 et de 2,4**

La matière première utilisée, lot F, est un lot de N-acétylhéparosane préparé selon la méthode décrite dans l'Exemple 2.

La détermination de la répartition des masses moléculaires des chaînes qui constituent ce composé a été réalisée par chromatographie d'exclusion selon la méthode décrite dans l'Exemple 1.

L'examen du profil permet de constater que le lot F contient 92 % en masse de chaînes dont la masse moléculaire est comprise entre 1500 et 15000 Da.

Le pic majoritaire est situé à environ 4800 Da. Une fraction pondérale du lot F au moins égale à 80 % possède une masse moléculaire comprise entre 2400 et 10000 Da.

Le taux d'acide uronique de ce produit est de 2,4 µmoles/mg.

Etape a- Désacétylation partielle

On procède comme il est décrit dans l'Exemple 5, (Etape a) en utilisant 2,5 g du lot F et 50 ml de NaOH 2N.

On obtient 1,6 g de produit N-désacétylé à 80 %.

Le pourcentage de la N-désacétylation a été évalué par le dosage des groupes amino libres.

Etape b et c - N-sulfatation et formation du sel de tétrabutylammonium

Ces deux étapes sont identiques aux Etapes b et c décrites dans l'Exemple 5.

On obtient 4,7 g de sel de tétrabutylammonium.

Etape d- O-sulfatation

On dissout 2,9 g de sel obtenu ci-dessus dans 290 ml de formamide et on ajoute 17,4 g de complexe trioxyde de soufre-pyridine.

On procède comme il est décrit dans l'Etape d, de l'Exemple 5.

Le culot obtenu après la deuxième centrifugation est dissout dans l'eau ultra-purifiée, puis on dialyse contre de l'eau ultra-purifiée et on lyophilise.

On obtient ainsi 3,68 g de produit.

Etape e- Gel-filtration

Le produit obtenu à l'étape précédente est mis en solution dans une solution de NaCl 0,5 M puis il est déposé sur une colonne Séphacryl S 300 HR équilibrée par du NaCl 0,5 M. L'effluent est recueilli par un collecteur de fractions.

Les fractions ayant une masse moléculaire comprise entre 1400 Da et 10000 Da sont regroupées et concentrées au Rotavapor. On précipite par 4 volumes d'éthanol, on centrifuge, on reprend le précipité à l'eau ultra-purifiée, on dialyse la solution, ainsi obtenue, extensivement contre de l'eau ultra-purifiée, on lyophilise et on sèche.

On obtient ainsi 1,6 g d'un héparosane-N,O-sulfaté appelé lot F1.

Les fractions correspondant à des masses moléculaires comprises entre 5000 Da et 35000 Da sont rassemblées, concentrées au Rotavapor et à la solution ainsi obtenue, on ajoute 4 volumes d'éthanol, on centrifuge, on reprend le précipité à l'eau, on dialyse la solution ainsi obtenue contre de l'eau ultra-purifiée, et on lyophilise. Le lyophilisat est soumis à un nouveau fractionnement selon la méthode indiquée au début de cette étape.

La répartition des masses moléculaires des différentes fractions est déterminée par chromatographie d'exclusion selon la méthode décrite dans l'Exemple 1. On regroupe les fractions qui contiennent des héparosanes-N,O-sulfatés ayant une masse moléculaire comprise entre 2000 et 26000 Da. On précipite par 4 volumes d'éthanol, on centrifuge et on re-dissout le culot dans l'eau distillée, on dialyse et on lyophilise. On obtient ainsi 0,6 g d'un héparosane-N,O-sulfaté appelé lot F2.

Les caractéristiques des lots F1 et F2 sont indiquées dans le tableau XV. Des résultats déduits des profils d'élution sont rassemblés dans le tableau XVI.

## TABLEAU XV

### Caractéristiques des héparosanes-N,O-sulfatés correspondant aux lots F1 et F2.

| | Taux d'acide uronique (µmol/mg) | Taux de $NH_2$ (µmol/mg) | Taux de désacétylation | Taux de sulfatation (rapport sulfate /carboxyle) |
|---|---|---|---|---|
| **Lot F1** | 1,58 | < 0,01 | 80 % | 2,4 |
| **Lot F2** | 1,58 | < 0,01 | 80 % | 2,25 |

## TABLEAU XVI

### Répartition des masses moléculaires des produits correspondant aux lots F1 et F2

| | PMo | $PM_1$ | $PM_2$ | $PM_3$ |
|---|---|---|---|---|
| **Lot F1** | 10700 | 7700 | 5800 | 3300 |
| **Lot F2** | 24400 | 16325 | 8600 | 6900 |

La définition de PMo, $PM_1$, $PM_2$ et $PM_3$ est identique à celle indiquée dans le tableau III.

L'héparosane-N,O-sulfaté du lot F1 contient environ 99 % en masse de chaînes ayant des masses moléculaires comprises entre 1500 et 15000 Da et celui du lot F2 contient environ 84,6 % en masse de chaînes de masses moléculaires de 1500 à 15000 Da, et environ 70 % en masse de chaînes de masses moléculaires de 6900 à 13500 Da.

Spectre de résonance magnétique nucléaire (RMN)

Des spectres RMN du proton et du carbone $^{13}C$ sont obtenus avec l'héparosane-N,O-sulfaté du lot F1 (Spectres réalisés sur AMX 500 solvant $D_2O$).

L'étude du spectre du proton et notamment du rapport d'intensité des protons des sucres sur les protons d'amines désacétylées conduit à proposer environ 20 % de motifs glucosamine N-acétylé.

L'étude du spectre $^{13}C$ confirme que le motif glucosamine est bien N-sulfaté. L'acide glucuronique, dans la majorité des structures disaccharidiques n'est pas O-sulfaté en position 2 et 3.

**PREPARATIONS**

**PREPARATION A**

**Préparation d'un N-acétylhéparosane majoritairement de haute masse moléculaire (Procédé I)**

**1) Culture de la souche *Escherichia coli* (K5) et séparation d'un filtrat contenant du N-acétylhéparosane**

On ensemence 400 ml du milieu D, de composition précisée dans le tableau XVII ci-après, avec la souche *Escherichia coli* SEBR 3282 et on incube sous agitation pendant 2 h à 37°C.

La préculture obtenue est alors transférée dans un fermenteur de 18,5 l contenant 11 l du milieu C, de composition précisée aussi dans le tableau XVII ci-après, et on incube pendant 6 h 30 min à 37°C et pH égal à 7,2, la pression partielle en oxygène étant maintenue à 40 mmHg par régulation de l'injection d'air (jusqu'à 20 l/min) et de l'agitation. On ajoute alors du glycérol en introduisant de façon continue une solution stérile contenant 500 g/l de glycérol à raison de 18 g/h pendant 16-17 heures.

On poursuit la culture dans les mêmes conditions de température, de pH et de pression partielle en oxygène jusqu'à consommation quasi totale du glycérol. Le suivi de la DO (λ = 600 nm) de la suspension de culture après la fin de l'ajout en glycérol, montre un état stationnaire ou de lyse légère jusqu'à l'arrêt de la culture à 28-30 h d'âge en fermenteur.

On refroidit alors le bouillon de culture à 25°C puis on le filtre à travers une membrane de porosité 0,22 µm. On obtient ainsi environ 12 l de filtrat contenant du N-acétylhéparosane majoritairement de haute masse moléculaire.

**TABLEAU XVII**

Composition et préparation du milieu C et du milieu D.

**MILIEU C**

Dans 900 ml d'eau ultra-purifiée dissoudre dans l'ordre :

| | |
|---|---|
| NTA (acide nitrilotriacétique) | 1000 mg |
| $K_2HPO_4$ | 790 mg |
| Acide glutamique | 11000 mg |
| $MgCl_2$, $6H_2O$ | 500 mg |
| $K_2SO_4$ | 450 mg |
| $FeSO_4$, $7H_2O$ | 18 mg |
| $CaCl_2$, $2H_2O$ | 2 mg |
| NaCl | 500 mg |
| KCl | 5000 mg |
| Solution d'oligo-éléments (cf Tableau II, Exemple 1) | 1 ml |
| Glycérol | 10000 mg |

Ajuster le pH à 7,2 avec de la potasse concentrée de densité 1,38 et compléter à 1000 ml avec de l'eau ultra-purifiée. Effectuer une filtration stérilisante sur membrane de 0,2 µm.

Solution de glycérol

Dissoudre 50 g de glycérol dans une quantité adéquate d'eau ultra-purifiée et ajuster le volume à 1000 ml avec le même solvant. Effectuer une filtration stérilisante sur membrane de 0,2 µm. L'anti-mousse employé en cours de fermentation est le Struktol J 673 (Schill et Seilacher®).

**MILIEU D**

La préparation du milieu D est identique à celle du milieu C, à la différence prés, qu'il convient d'ajouter en plus le tampon (pH 7,2) : acide 3-morpholinopropanesulfonique après addition de l'agent anti-mousse.

**2) Isolement et purification d'un N-acétylhéparosane majoritairement de haute masse moléculaire**

Le N-acétylhéparosane majoritairement de haute masse moléculaire à été isolé et purifié selon le procédé décrit dans l'Exemple 2 [ 2) Isolement et purification d'un N-acétylhéparosane majoritairement de faible masse moléculaire, Etape a - Etape f].

**3) Caractérisation du N-acétylhéparosane obtenu**

Détermination de la répartition des masses moléculaires par chromatographie d'exclusion

Compte-tenu des étalons actuellement utilisés, la répartition des masses moléculaires a été évaluée de manière approximative.

Le N-acétylhéparosane obtenu pour cette préparation est composé de chaînes de masses moléculaires comprises entre 20000 Da et 500000 Da, et la masse moyenne se situe à environ 100000-200000 Da.

Dosage des acides uroniques

Le taux d'acide uronique du produit purifié à l'issue de l'étape finale est de 2,2 µmoles/mg.

Spectrophotométrie dans le domaine de l'ultra-violet et visible

Le spectre obtenu permet d'affirmer que ce lot contient moins de 0,5 % d'ADN.

Dosage des protéines totales

Le taux de protéines totales est inférieur à 0,5 %

Dosage des groupes amino libres $NH_2$

Le taux de $NH_2$ est inférieur à 0,1 µmole/mg.

**PREPARATION B**

**Préparation d'un N-acétylhéparosane majoritairement de haute masse moléculaire (Procédé II)**

**1) Culture de la souche Escherichia coli (K5)**

La culture de la souche Escherichia coli SEBR 3282 a été réalisée selon la méthode décrite dans la Préparation A. On obtient environ 12 l de culture contenant du N-acétylhéparosane majoritairement de haute masse moléculaire.

**2) Purification préliminaire**

On procède comme il est décrit dans l'Exemple 3 [ 2) Purification préliminaire] en utilisant au stade d, une cartouche à fibres creuses Amicon®, seuil de coupure 30000 Da ou équivalent.

**3) Isolement et purification d'un N-acétylhéparosane majoritairement de haute masse moléculaire :**

On procéde comme il est indiqué dans l'Exemple 3 [ 3) Isolement et purification d'un N-acétylhéparosane majoritairement de faible masse moléculaire].

**PREPARATION C**

**Modifications chimiques du N-acétylhéparosane majoritairement de haute masse moléculaire issu de la Préparation A**

Comme matière première pour les différentes modifications chimiques, on utilise un N-acétylhéparosane appelé lot G1 préparé selon le procédé décrit dans la Préparation A.

Le taux d'acide uronique de ce produit est de 2,41 µmoles/mg.

Etape a- Désacétylation partielle

On dissout 1,9 g du lot G1 dans 38,5 ml de NaOH 2N. On porte la solution à 50°C et on laisse réagir à cette température pendant 8 heures sous azote.

On ajuste ensuite le pH à 8,25 par addition de HCl 2N. On dialyse contre de l'eau ultra-purifiée puis on lyophilise.

On obtient ainsi 1,6 g de produit.

Taux de N-désacétylation

Le dosage des groupes amino-libres ($NH_2$) indique que le N-acétylhéparosane a été N-désacétylé à 80 %.

Etape b - N-sulfatation

On dissout 1,3 g du produit obtenu à l'étape précédente dans 57 ml d'eau ultra-purifiée, on ajoute 1,9 g de $Na_2CO_3$ et 1,9 g de complexe trioxyde de soufre-triméthylamine et on porte à 55°C pendant 20 heures. On ajuste ensuite la conductivité de la solution à celle d'une solution de NaCl 0,5 M par addition d'eau déminéralisée et on précipite par 4 volumes d'éthanol. On centrifuge, on reprend le culot avec une solution de NaCl 0,5 M, on précipite par 4 volumes d'éthanol et on centrifuge.

On dissout le culot dans l'eau ultra-purifiée et on dialyse avec de l'eau ultra-purifiée selon le procédé décrit dans l'Exemple 1 puis, on lyophilise.

On obtient ainsi 1,809 g d'héparosane N-sulfaté.

Etape c - Formation du sel de tétrabutylammonium

On dissout 800 mg du produit préparé à l'étape précédente dans 100 ml d'eau. On dépose cette solution sur une colonne d'échange d'ions Dowex 50 W 8 et on procède comme il est décrit dans l'Exemple 4 [ 1 ) Modifications chimiques du lot B, Etape b].

Après lyophilisation on obtient environ 1,3 g de sel.

Etape d - O-sulfatation

On dissout le sel obtenu ci-dessus dans 80 ml de formamide, on ajoute 5,6 g de complexe trioxyde de soufre-pyridine. On laisse réagir 6 heures à 30°C puis on ajoute 16 ml de NaCl 2 M. On porte à pH 7 et on précipite par 2 volumes d'éthanol. On reprend le précipité avec une solution de NaCl 0,5 M, on re-précipite avec 2 volumes d'éthanol, on dialyse contre de l'eau ultra-purifiée et on concentre au Rotavapor.

Etape e- Gel-filtration

La solution concentrée obtenue à l'étape précédente est fractionnée par gel filtration en utilisant une colonne Sephacryl S 300 HR et en utilisant comme éluant une solution de NaCl 0,5 M.

On isole les fractions qui correspondent à des masses moléculaires comprises entre 10000 et 500000 Da.

On ajoute 2 volumes d'éthanol puis on concentre. On ajuste ensuite la conductivité à celle d'une solution de NaCl 0,5 M en ajoutant de l'eau.

On répète le fractionnement par gel filtration et on recueille les fractions contenant un héparosane-N,O-sulfaté constitué de chaînes ayant des masses moléculaires moyennes de 100000 à 200000 Da, comme aussi les fractions contenant un héparosane-N,O-sulfaté constitué de chaînes ayant des masses moléculaires moyennes d'environ 50000 Da et d'environ 12000 Da.

A chacune de ces trois fractions, on ajoute 5 volumes d'éthanol, on les soumet à une dialyse contre de l'eau ultra-purifiée et on soumet les solutions obtenues après dialyse, à une lyophilisation.

On obtient ainsi 3 lots d'héparosane-N,O-sulfatés :

- le lot G1 est un héparosane-N,O-sulfaté constitué de chaînes ayant une masse moléculaire moyenne de 100000 à 200000 Da. On isole 0,140 g de ce lot.
- le lot G2 est un héparosane-N,O-sulfaté constitué de chaînes ayant une masse moléculaire moyenne d'environ 50000 Da. On isole 0,350 g de cet héparosane-N,O-sulfaté.
- le lot G3 est un héparosane-N,O-sulfaté constitué de chaînes ayant une masse moléculaire moyenne de 12000 Da. On isole environ 0,100 g de ce dernier lot.

Les caractéristiques des trois lots d'héparosane-N,O-sulfaté décrits dans cet exemple sont rassemblés dans le tableau XVIII.

## TABLEAU XVIII

### Caractéristiques des héparosanes-N,O-sulfatés correspondant aux lots G1, G2 et G3

| | Taux d'acide uronique (µmol/mg) | Taux de $NH_2$ (µmol/mg) | Taux de désacétylation | Taux de sulfatation (rapport sulfate /carboxyle) |
|---|---|---|---|---|
| **Lot G1** | 1,48 | < 0,01 | 80 % | 2,6 |
| **Lot G2** | 1,45 | < 0,01 | 80 % | 2,6 |
| **Lot G3** | 1,45 | < 0,01 | 80 % | 2,6 |

**Revendications**

**1.** Héparosanes-N,O-sulfatés constitués par des chaînes ou par un mélanae de chaînes de masse moléculaire entre 1500 et 15000 Da, ayant la structure disaccharidique répétée de formule I :

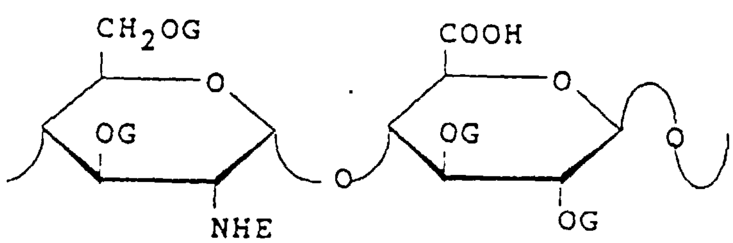

(I)

dans laquelle,

- E représente, dans 0 à 80 % des unités disaccharidiques desdits héparosanes-N,O-sulfatés, un groupe acétyle, et dans les unités disaccharidiques restantes, un groupe sulfate et éventuellement un atome d'hydrogène,
- G représente un atome d'hydrogène et un groupe sulfate, le degré de sulfatation, exprimé comme le rapport sulfate/carboxyle, étant compris entre 1,5 et 3,0, et les sels pharmaceutiquement acceptables desdits héparosanes-N,O-sulfatés.

**2.** Héparosanes-N,O-sulfatés selon la revendication 1, caractérisés en ce que E représente un groupe acétyle et un groupe sulfate.

**3.** Héparosanes-N,O-sulfatés selon la revendication 1, constitués par des chaines ou par un mélange de chaînes de masse moléculaire entre 1500 et 15000 Da, ayant la structure disaccharidique répétée de formule I :

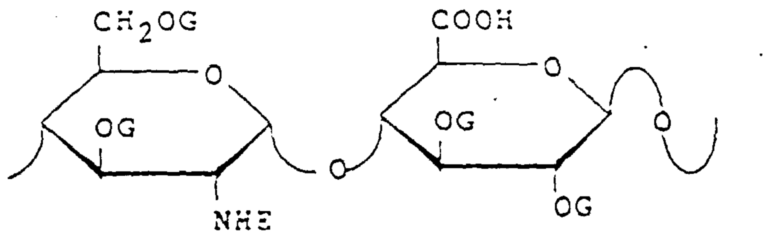

(I)

dans laquelle,

- E représente, dans 0 à 80 % des unités disaccharidiques desdits héparosanes-N,O-sulfatés, un groupe acétyle, et dans les unités disaccharidiques restantes, un groupe sulfate et éventuellement un atome d'hydrogène,
- G représente un atome d'hydrogène et un groupe sulfate,

le degré de sulfatation, exprimé comme rapport sulfate/carboxyle étant de 1,5 à 3,0,
les deux extrémités, réductrice et non réductrice des chaînes desdits héparosanes-N,O-sulfatés étant des unités uroniques sulfatées ou non, de la glucosamine sulfatée ou non, ou de la N-acétyl glucosamine, sulfatée ou non,
et les sels pharmaceutiquement acceptables desdits héparosanes-N,O-sulfatés.

4. Héparosanes-N,O-sulfatés selon l'une quelconque des revendications 1 à 3,caractérisés en ce que E représente, dans 0 à 60 % des unités disaccharidiques desdits héparosanes-N,O-sulfatés, un groupe acétyle.

5. Héparosanes-N,O-sulfatés selon l'une quelconque des revendications 1 à 4, caractérisés en ce qu'ils comportent au moins 90 % en masse de chaînes de masse moléculaire inférieure à 11000 Da.

6. Héparosanes-N,O-sulfates selon la revendication 1, caractérisés en ce qu'ils contiennent moins de 0,2 µmol/mg de groupe amino ($NH_2$).

7. Héparosanes-N,O-sulfatés selon l'une quelconque des revendications 1 à 4, constitués de chaînes de masse moléculaire moyenne de 4000 Da à 7000 Da et ayant un degré de sulfatation exprimé comme le rapport sulfate/carboxyle compris entre 1,7 et 3.

8. Héparosanes-N,O-sulfatés selon la revendication 1 caractérisés en ce qu'ils sont constitués par au moins 70 % en masse de chaînes de masses moléculaires comprises entre 5000 et 7000 Da, en ce que le degré de sulfatation exprimé comme le raooort sulfate/caboxyle est compris entre 1,8 et 2,5 et en ce que E représente, dans 0 à 20 % des unités disaccharidiques desdits héparoanes-N,O-sulfatés, un groupe acétyle.

9. Héparosanes-N,O-sulfatés selon la revendication 1 caractérisés en ce qu'ils sont constitués par au moins 70 % en masse de chaînes de masses moléculaires comprises entre 10000 et 12000 Da, en ce que le degré de sulfatation exprimé comme le rapport sulfate/carboxyle est compris entre 1,8 et 2,5, et en ce que E représente, dans 0 à 20 % des unités disaccharidiques desdits héparosanes-N,O-sulfatés, un groupe acétyle.

10. Héparosanes-N,O-sulfatés selon la revendication 1 caractérisés en ce qu'ils sont constitués par au moins 70 % en masse de chaînes de masses moléculaires comprises entre 6000 et 8000 Da, en ce que le degré de sulfatation exprimé comme le rapport sulfate/carboxyle est compris entre 2,0 et 2,8, et en ce que E représente, dans 0 à 60 % des unités disaccharidiques desdits héparosanes-N,O-sulfatés, un groupe acétyle.

11. Héparosanes-N,O-sulfatés -sulfatés selon la revendication 1 caractérisés en ce qu'ils sont constitués par au moins 80 % en masse de chaînes de masses moléculaires comprises entre 2300 et 7200 Da, en ce que le degré de sulfatation exprimé comme le rapport sulfate/carboxyle est compris entre 1,8 et 2,5, et en ce que E représente, dans 0 à 20 % des unités disaccharidiques desdits héparosanes-N,O-sulfatés, un groupe acétyle.

12. Héparosanes-N,O-sulfatés selon la revendication 1 caractérisés en ce qu'ils sont constitués par au moins 80 % en masse de chaînes de masses moléculaires comprises entre. 3300. et 7700 Da, en ce que le degré de sulfatation exprimé comme le rapport sulfate/carboxyle est compris entre 1,8 et 2,5, et en ce que E représente, dans 0 à 20

% des unités disaccharidiques desdits héparosanes-N,O-sulfatés, un groupe acétyle.

**13.** Héparosanes-N,O-sulfatés selon la revendication 1 caractérisés en ce qu'ils sont constitués par au moins 70 % en masse de chaînes de masses moléculaires comprises entre 6900 et 13500 Da, en ce que le degré de sulfatation exprimé comme le rapport sulfate/carboxyle est compris entre 1,8 et 2,5, et en ce que E représente, dans 0 à 20 % des unités disaccharidiques desdits héparosanes-N,O-sulfatés,un groupe acétyle.

**14.** Héparosanes-N,O-sulfatés selon la revendication 1 caractérisés en ce qu'ils sont constitués par au moins 80 % en masse de chaînes de masses moléculaires comprises entre 4000 et 10300 Da, en ce que le degré de sulfatation exprimé comme le rapport sulfate/carboxyle est compris entre 2,0 et 2,8, et en ce que E représente,dans 0 à 60 % des unités disaccharidiques desdits héparosanes-N,O-sulfatés,un groupe acétyle

**15.** Composition d'héparosane-N,O-sulfaté caractérisée en ce qu'elle contient au moins 70 % en masse d'un héparosane-N,O-sulfaté selon l'une quelconque des revendications 1 à 14.

**16.** Procédé de préparation d'une composition contenant 70 % à 100 % d'un héparosane-N,O-sulfaté selon la revendication 1, caractérisé en ce qu'il comprend la séquence des étapes suivantes :

- étape a : culture d'une souche d'Escherichia coli (K5),
- étape b : isolement et purification - précédé ou non d'une purification préliminaire du N-acétylhéparosane formé pour obtenir une composition contenant 70 % à 100 % d'un N-acétylhéparosane constitué par des chaînes ou par un mélange de chaînes de masse moléculaire entre 1500 et 15000 Da, ayant la structure disaccharidique répétée de formule II :

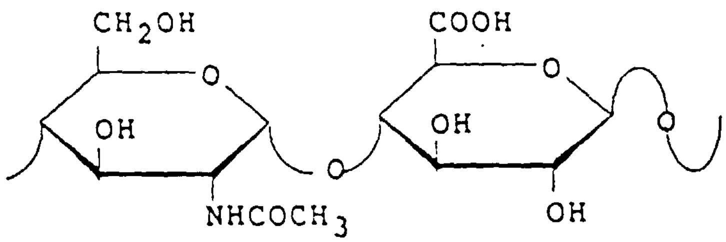

(II)

- étape c : désacétylation partielle de cette composition de N-acétylhéparosane pour obtenir une composition contenant 70 % à 100 % d'un héparosane constitué par des chaînes ou par un mélange de chaînes de masse moléculaire entre 1500 et 15000 Da ayant la structure disaccharidique répétée de formule III :

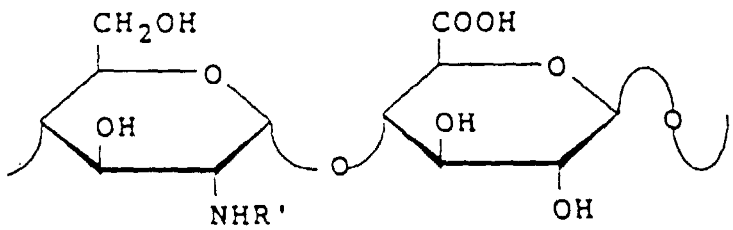

(III)

dans laquelle R' représente, dans 0 à 80 % des unités disaccharidiques, un groupe acétyle, et dans les unités disaccharidiques restantes un atome d'hydrogène,

- étape d :
  - soit N,O-sulfatation partielle de cette composition d'héparosane,
  - soit N,O-sulfatation partielle de cette composition d'héparosane suivie d'une étape de N-sulfatation totale,
  - soit une N-sulfatation totale ou partielle suivie d'une étape de O-sulfatation totale ou partielle,

et comporte éventuellement une ou plusieurs étapes de fractionnement des masses moléculaires effectuées à la fin des étapes a, b. c ou d.

**17.** Procédé selon la revendication 16 caractérisé en ce que la souche d'Escherichia coli (K5) est la souche SEBR 3282 (déposée auprès de la CNCM de l'Institut Pasteur, Paris, France, sous le N° 1-1013) ou un mutant de cette souche, soit spontané soit induit.

**18.** Procédé selon la revendication 16 caractérisé en ce que la culture de la souche d'Escherichia coli (K5) est continuée au moins deux heures après l'arrêt de la croissance de la biomasse.

**19.** Procédé selon la revendication 16, caractérisé en ce que l'isolement et la purification du N-acétylhéparosane, contiennent au moins une étape de précipitation alcoolique, et au moins une étape de chromatographie d'échange d'ions.

**20.** Procédé selon la revendication 16, caracterisé en ce que l'isolement et la purification du N-acétylhéparosane sont réalisés à l'aide d'un procédé comportant la séquence des étapes suivantes :

- étape $a_1$ : Précipitation par l'éthanol,
- étape $b_1$ : Dialyse,
- étape $c_1$ : Précipitation par l'éthanol, puis déshydratation et séchage,
- étape $d_1$ : Purification par chromatographie d'échange d'anions,
- étape $e_1$ : Précipitation par l'éthanol de l'éluat obtenu par l'étape $d_1$, déshydratation, séchage et broyage,

dans lequel une des étapes $a_1$ ou $c_1$ peut être supprimée.

**21.** Procédé selon la revendication 16, caractérisé en ce que l'isolement et la purification du N-acétylhéparosane sont réalisés à l'aide d'un procédé comportant la séquence des étapes suivantes :

- étape $a'_1$ : Oialyse,
- étape $b'_1$ : Purification en milieu acide, élimination des impuretés insolubles dans des solutions aqueuses de pH 3,5 et pH 1,8,
- étape $c'_1$ : Précipitation par l'éthanol, puis déshydratation et séchage,
- étape $d'_1$ : Hydrolyse alcaline et dialyse,
- étape $e'_1$ : Purification par chromatographie d'échange d'anions,
- étape $f'_1$ : Purification par chromatographie d'exclusion.

**22.** Procédé selon la revendication 16, caractérisé en ce que le N-acétylhéparosane obtenu de la culture d'une souche d'Escherichia coli (K5) est soumis avant l'isolement et la purification à une purification préliminaire, réalisée à l'aide d'un procédé comportant la séquence des étapes suivantes :

- étape $a''_1$ : Centrifugation de la suspension obtenue à la fin de la culture,
- étape $b''_1$ : Mise en contact du surnageant avec une solution alcaline,
- étape $c''_1$ : Préfiltration,
- étape $d''_1$ : Concentration sur membrane de seuil de coupure déterminé,
- étape $e''_1$ : Dialyse,

dans lequel l'étape $e''_1$ peut être supprimée.

**23.** Procédé selon la revendication 16 caractérisé en ce que l'étape de N,O-sulfatation partielle est suivie d'une N-sulfatation totale, réalisée par un complexe d'anhydride sulfurique avec une base organique, dans un solvant aqueux de pH basique.

**24.** Procédé selon la revendication 16 caractérisé en ce que l'étape de N-sulfatation totale est suivie d'un fractionnement des masses moléculaires.

**25.** Composition pharmaceutique contenant comme principe actif un héparosane-N,O-sulfaté selon l'une quelconque des revendications 1 à 14, en association ou en mélange avec un excipient inerte, pharmaceutiquement acceptable.

**26.** Composition pharmaceutique contenant comme principe actif une composition d'héparosane-N,O-sulfaté selon la revendication 15, en association ou en mélange avec un excipient inerte, pharmaceutiquement acceptable.

**27.** Composition pharmaceutique selon l'une quelconque des revendications 25 et 26 utilisable pour la régulation de la coagulation.

**28.** Héparosanes constitués par un mélange de chaînes de masse moléculaire entre 1500 et 15000 Da, ayant la structure disaccharidique répétée de formule III :

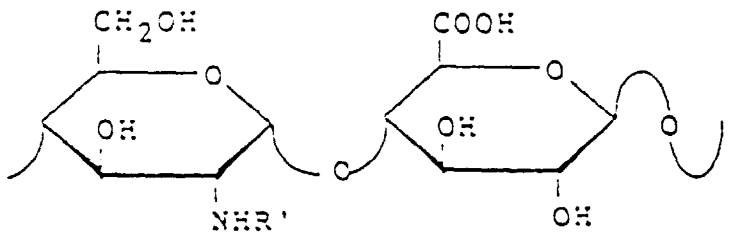

(III)

dans laquelle R' représente, dans 0 à 80 % des unités disaccharidiques, un groupe acétyle, et dans les unités disaccharidiques restantes un atome d'hydrogène.

**29.** Héparosanes selon la revendication 28, caractérisés en ce que R' représente, dans 0 à 60 % des unités disaccharidiques desdits héparosanes, un groupe acétyle.

**30.** Composition d'héparosane caractérisée en ce qu'elle contient au moins 70 % en masse d'un héparosane selon l'une quelconque des revendications 28 et 29.

## Patentansprüche

**1.** N,O-sulfatierte Heparosane, die aus Ketten oder einer Mischung von Ketten mit einer Molekularmasse zwischen 1500 und 15000 Da aufgebaut sind, mit einer sich wiederholenden Disaccharidstruktur der Formel I:

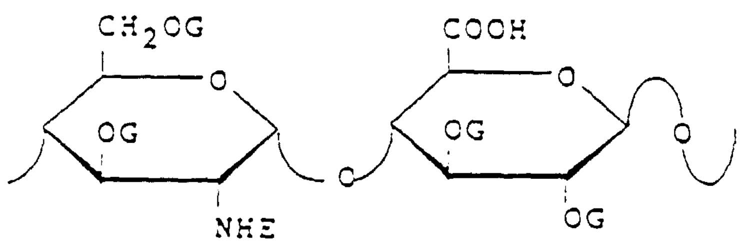

(I)

in der

- E bei 0 bis 80 % der Disaccharideinheiten der genannten N,O-sulfatierten Heparosane eine Acetylgruppe und bei den verbleibenden Disaccharideinheiten eine Sulfatgruppe und gegebenenfalls ein Wasserstoffatom darstellt,
- G ein Wasserstoffatom und eine Sulfatgruppe darstellt, wobei der Sulfatierungsgrad, als Sulfat/Carboxyl-Verhältnis ausgedrückt, zwischen 1,5 und 3,0 liegt, sowie die pharmazeutisch annehmbaren Salze dieser N,O-sulfatierten Heparosane.

**2.** N,O-sulfatierte Heparosane nach Anspruch 1, **dadurch gekennzeichnet,** daß E eine Acetylgruppe und eine Sulfatgruppe bedeutet.

**3.** N,O-sulfatierte Heparosane nach Anspruch 1, welche aus Ketten oder einer Mischung von Ketten mit einer Molekülmasse zwischen 1500 und 15000 Da aufgebaut sind, mit einer sich wiederholenden Disaccharidstruktur der Formel I:

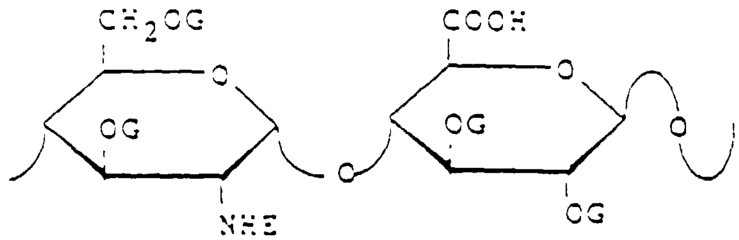

(I)

in der

- E bei 0 bis 80 % der Disaccharideinheiten der genannten N,O-sulfatierten Heparosane eine Acetylgruppe und bei den verbleibenden Disaccharideinheiten eine Sulfatgruppe und gegebenenfalls ein Wasserstoffatom und
- G ein Wasserstoffatom und eine Sulfatgruppe bedeuten,

wobei der als Sulfat/Carboxyl-Verhältnis ausgedrückte Sulfatierungsgrad 1,5 bis 3,0 beträgt und die beiden reduzierenden und nichtreduzierenden Enden der Ketten der genannten N,O-sulfatierten Heparosane gegebenenfalls sulfatierte Uroneinheiten. Einheiten des gegebenenfalls sulfatierten Glucosamins oder Einheiten des gegebenenfalls N-Acetylglucosamins sind.
und die pharmazeutisch annehmbaren Salze dieser N,O-sulfatierten Heparosane.

**4.** N,O-sulfatierte Heparosane nach einem der Ansprüche 1 bis 3, **dadurch gekennzeichnet**, daß E bei 0 bis 60 % der Disaccharideinheiten der genannten N,O-sulfatierten Heparosane eine Acetylgruppe bedeutet.

**5.** N,O-sulfatierte Heparosane nach einem der Ansprüche 1 bis 4, **dadurch gekennzeichnet**, daß sie mindestens 90 Massen-% von Ketten mit einer Molekularmasse von weniger als 11000 Da aufweisen.

**6.** N,O-sulfatierte Heparosane nach Anspruch 1, **dadurch gekennzeichnet,** daß sie mindestens 0.2 µMol/mg Aminogruppen ($NH_2$) aufweisen.

**7.** N,O-sulfatierte Heparosane nach irgendeinem der Ansprüche 1 bis 4, welche aus Ketten mit einer mittleren Molekülmasse von 4000 Da bis 7000 Da aufgebaut sind und einen als Sulfat/Carboxyl-Verhältnis ausgedrückten Sulfatierungsgrad zwischen 1,7 und 3 aufweisen.

**8.** N,O-sulfatierte Heparosane nach Anspruch 1, **dadurch gekennzeichnet**, daß sie zu mindestens 70 Massen-% aus Ketten mit Molekülmassen zwischen 5000 und 7000 Da aufgebaut sind und der als Sulfat/Carboxyl-Verhältnis ausgedrückte Sulfatierungsgrad zwischen 1.8 und 2.5 liegt und E bei 0 bis 20 % der Disaccharideinheiten der genannten N.O-sulfatierten Heparosane eine Acetylgruppe darstellt.

**9.** N,O-sulfatierte Heparosane nach Anspruch 1, **dadurch gekennzeichnet**, daß sie zu mindestens 70 Massen-% aus Ketten mit Molekülmassen zwischen 10000 und 12000 Da aufgebaut sind und der als Sulfat/Carboxyl-Verhältnis ausgedrückte Sulfatierungsgrad zwischen 1.8 und 2,5 liegt und E bei 0 bis 20 % der Disaccharideinheiten der genannten N,O-sulfatierten Heparosane eine Acetylgruppe darstellt.

**10.** N,O-sulfatierte Heparosane nach Anspruch 1, **dadurch gekennzeichnet**, daß sie zu mindestens 70 Massen-% aus Ketten mit Molekülmassen zwischen 6000 und 8000 Da aufgebaut sind, der als Sulfat/Carboxyl-Verhältnis ausgedrückte Sulfatierungsgrad zwischen 2,0 und 2,8 liegt und E bei 0 bis 60 % der Disaccharideinheiten der genannten N,O-sulfatierten Heparosane eine Acetylgruppe bedeutet.

**11.** N,O-sulfatierte Heparosane nach Anspruch 1, **dadurch gekennzeichnet**, daß sie zu mindestens 80 Massen-% aus Ketten mit Molekülmassen zwischen 2300 und 7200 Da aufgebaut sind, der als Sulfat/Carboxyl-Verhältnis ausgedrückte Sulfatierungsgrad zwischen 1,8 und 2,5 liegt und E bei 0 bis 20 % der Disaccharideinheiten der genannten N,O-sulfatierten Heparosane eine Acetylgruppe darstellt.

**12.** N,O-sulfatierte Heparosane nach Anspruch 1, **dadurch gekennzeichnet**, daß sie zu mindestens 80 Massen-% aus Ketten mit Molekülmassen zwischen 3300 und 7700 Da aufgebaut sind, der als Sulfat/Carboxyl-Verhältnis ausgedrückte Sulfatierungsgrad zwischen 1,8 und 2,5 liegt und E bei 0 bis 20 % der Disaccharideinheiten der

genannten N,O-sulfatierten Heparosane eine Acetylgruppe darstellt.

**13.** N,O-sulfatierte Heparosane nach Anspruch 1, **dadurch gekennzeichnet**, daß sie zu mindestens 70 Massen-% aus Ketten mit Molekülmassen zwischen 6900 und 13500 Da aufgebaut sind, der als Sulfat/Carboxyl-Verhältnis ausgedrückte Sulfatierungsgrad zwischen 1,8 und 2,5 liegt und E bei 0 bis 20 % der Disaccharideinheiten der genannten N,O-sulfatierten Heparosane eine Acetylgruppe darstellt.

**14.** N,O-sulfatierte Heparosane nach Anspruch 1, **dadurch gekennzeichnet**, daß sie zu mindestens 80 Massen-% aus Ketten mit Molekülmassen zwischen 4000 und 10300 Da aufgebaut sind, der als Sulfat/Carboxyl-Verhältnis ausgedrückte Sulfatierungsgrad zwischen 2,0 und 2,8 liegt und E bei 0 bis 60 % der Disaccharideinheiten der genannten N,O-sulfatierten Heparosane eine Acetylgruppe darstellt.

**15.** Zubereitung auf der Grundlage von N.O-sulfatiertem Heparosan, **dadurch gekennzeichnet,** daß sie mindestens 70 Massen-% eines N,O-sulfatierten Heparosans nach einem der Ansprüche 1 bis 14 enthält.

**16.** Verfahren zur Herstellung einer Zubereitung, die 70 % bis 100 % eines N,O-sulfatierten Heparosans nach Anspruch 1 enthält, **dadurch gekennzeichnet,** daß es die Folge der nachstehenden Stufen umfaßt:

- Stufe a: Züchten eines Stammes von Escherichia coli (K5).
- Stufe b: nach einer eventuellen Vorreinigung - Isolierung und Reinigung des gebildeten N-Acetylheparosans zur Bildung einer Zusammensetzung, die 70 % bis 100 % eines N-Acetylheparosans enthält. welches aus Ketten oder einer Mischung von Ketten mit einer Molekülmasse zwischen 1500 und 15000 Da aufgebaut ist, mit einer sich wiederholenden Disaccharidstruktur der Formel II:

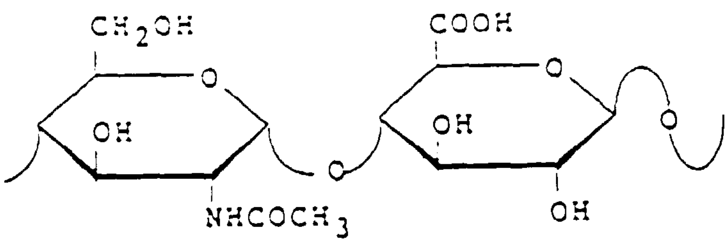

(II)

- Stufe c: teilweise Desacetylierung dieser N-Acetylheparosan-Zusammensetzung zur Bildung einer Zusammensetzung, die 70 % bis 100 % eines Heparosans enthält, welches aus Ketten oder einer Mischung von Ketten mit einer Molekülmasse zwischen 1500 und 15000 Da aufgebaut ist, mit einer sich wiederholenden Disaccharidstruktur der Formel III:

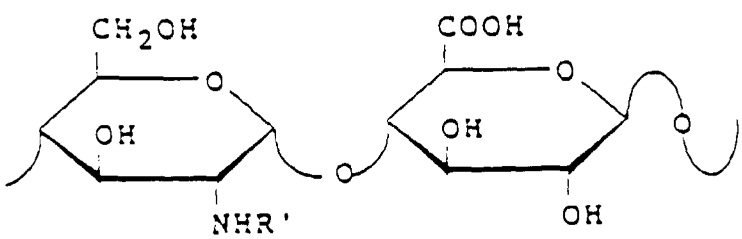

(III)

in der R' bei 0 bis 80 % der Disaccharideinheiten eine Acetylgruppe und bei den verbleibenden Disaccharideinheiten ein Wasserstoffatom bedeutet,
- Stufe d: - entweder teilweise N.O-Sulfatierung dieser Heparosan-Zusammensetzung,
  - oder teilweise N.O-Sulfatierung dieser Heparosan-Zusammensetzung, gefolgt von einer Stufe der vollständigen N-Sulfatierung,
  - oder eine vollständige oder teilweise N-Sulfatierung, gefolgt von einer Stufe der vollständigen oder teilweisen O-Sulfatierung,

und gegebenenfalls eine oder mehrere Stufen der Fraktionierung der Molekülmassen am Ende der Stufen a, b, c oder d.

**17.** Verfahren nach Anspruch 16, **dadurch gekennzeichnet**, daß der Stamm Escherichia coli (K5) der Stamm SEBR 3282 (der bei dem CNCM des Institut Pasteur, Paris, Frankreich, unter der Nr. 1-1013 hinterlegt worden ist) oder eine spontane oder induzierte Mutante dieses Stammes ist.

**18.** Verfahren nach Anspruch 16, **dadurch gekennzeichnet,** daß das Züchten des Stammes Escherichia coli (K5) während mindestens zwei Stunden nach Unterbrechung des Wachstums der Biomasse fortgesetzt wird.

**19.** Verfahren nach Anspruch 16, **dadurch gekennzeichnet**, daß die Isolierung und Reinigung des N-Acetylheparosans mindestens eine Stufe der alkoholischen Ausfällung und mindestens eine Stufe der Ionenaustauschchromatographie umfaßt.

**20.** Verfahren nach Anspruch 16, **dadurch gekennzeichnet**, daß die Isolierung und Reinigung des N-Acetylheparosans mit Hilfe eines Verfahrens durchgeführt werden, welches die Folge der nachstehenden Stufen umfaßt:

- Stufe $a_1$: Ausfällung mit Ethanol,
- Stufe $b_1$: Dialyse,
- Stufe $c_1$: Ausfällung mit Ethanol und dann Entwässerung und Trocknung.
- Stufe $d_1$: Reinigung durch Ionenaustauschchromatographie,
- Stufe $e_1$ : Ausfällung des in der Stufe $d_1$ erhaltenen Eluats mit Ethanol. Entwässerung. Trocknung und Vermahlen,

wobei auf eine der Stufe $a_1$ oder $c_1$ verzichtet werden kann.

**21.** Verfahren nach Anspruch 16, **dadurch gekennzeichnet**, daß die Isolierung und Reinigung des N-Acetylheparosans mit Hilfe eines Verfahrens durchgeführt werden. welches die Folge der nachstehenden Stufen umfaßt:

- Stufe $a'_1$: Dialyse
- Stufe $b'_1$: Reinigung in saurem Medium. Entfernen von in wäßrigen Lösungen mit pH-Werten von 3.5 und 1.8 unlöslichen Verunreinigungen.
- Stufe $c'_1$: Ausfällung mit Ethanol und dann Entwässerung und Trocknung,
- Stufe $d'_1$ : alkalische Hydrolyse und Dialyse.
- Stufe $e'_1$: Reinigung durch Ionenaustauschchromatographie.
- Stufe $f'_1$: Reinigung durch Ausschlußchromatographie.

**22.** Verfahren nach Anspruch 16, **dadurch gekennzeichnet**, daß das beim Züchten eines Stammes von Escherichia coli (K5) erhaltene N-Acetylheparosan vor der Isolierung und Reinigung einer vorausgehenden Reinigung unterworfen wird, welche mit Hilfe eines Verfahrens durchgeführt wird, das die Folge der nachstehenden Stufen umfaßt:

- Stufe $a''_1$ : Zentrifugieren der nach dem Züchten erhaltenen Suspension.
- Stufe $b''_1$ : Inkontaktbringen der überstehenden Flüssigkeit mit einer alkalischen Lösung.
- Stufe $c''_1$: Vorfiltration.
- Stufe $d''_1$ : Einengen auf einer Membran mit vorbestimmter Abtrennschwelle,
- Stufe $e''_1$: Dialyse,

wobei auf die Stufe $e''_1$ verzichtet werden kann.

**23.** Verfahren nach Anspruch 16, **dadurch gekennzeichnet**, daß die Stufe der teilweisen N,O-Sulfatierung von einer vollständigen N-Sulfatierung gefolgt wird, welche mit einem Schwefeltrioxidkomplex mit einer organischen Base in einem wäßrigen Lösungsmittel mit basischem pH-Wert durchgeführt wird.

**24.** Verfahren nach Anspruch 16, **dadurch gekennzeichnet**, daß die Stufe der vollständigen N-Sulfatierung von einer Fraktionierung der Molekülmassen gefolgt wird.

**25.** Pharmazeutische Zubereitung enthaltend als Wirkstoff ein N,O-sulfatiertes Heparosan nach einem der Ansprüche 1 bis 14 in Kombination oder in Mischung mit einem inerten, pharmazeutisch annehmbaren Trägermaterial.

**26.** Pharmazeutische Zubereitung enthaltend als Wirkstoff eine Zusammensetzung von N,O-sulfatiertem Heparosan nach Anspruch 15 in Kombination oder Mischung mit einem inerten, pharmazeutisch annehmbaren Trägermaterial.

**27.** Pharmazeutische Zubereitung nach irgendeinem der Ansprüche 25 und 25, geeignet für die Steuerung der Koagulation.

**28.** Heparosane gebildet aus einer Mischung von Ketten mit Molekülmassen zwischen 1500 und 15000 Da mit einer sich wiederholenden Disaccharidstruktur der Formel III:

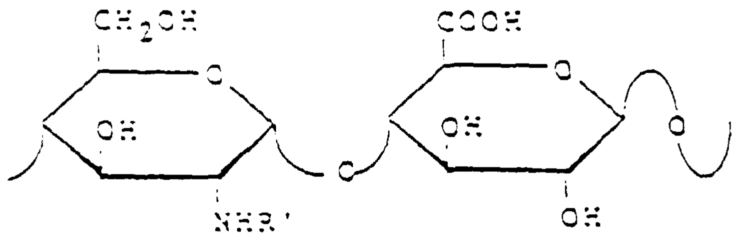

(III)

in der R' bei 0 bis 80 % der Disaccharideinheiten eine Acetylgruppe und bei den verbleibenden Disaccharideinheiten ein Wasserstoffatom bedeutet.

**29.** Heparosane nach Anspruch 28, **dadurch gekennzeichnet**, daß R' bei 0 bis 60 % der Disaccharideinheiten der genannten Heparosane eine Acetylgruppe darstellt.

**30.** Heparosan-Zubereitung, **dadurch gekennzeichnet**, daß sie mindestens 70 Massen-% eines Heparosans nach irgendeinem der Ansprüche 28 und 29 enthält.

## Claims

**1.** N,O-sulfated heparosanes comprising chains or a mixture of chains having a molecular weight of between 1500 and 15000 Da, having the repeating disaccharide structure of formula I :

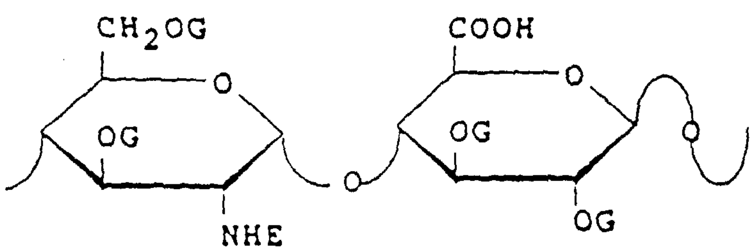

(I)

wherein

E represents an acetyl group in 0 to 80 % of the disaccharide units of said N,O-sulfated heparosanes and a sulphate group and optionally a hydrogen atom in the remaining disaccharide units,
G represents a hydrogen atom and a sulfate group, the degree of sulfation, expressed as the ratio of sulfate to carboxyl, being between 1.5 and 3.0, and the pharmaceutically acceptable salts of said N,O-sulfated heparosanes.

**2.** N,O-sulfated heparosanes according to claim 1, characterised in that E represents an acetyl group and a sulfate group.

**3.** N,O-sulfated heparosanes according to claim 1, comprising chains or a mixture of chains having a molecular weight of between 1500 and 15000 Da, having the repeating disaccharide structure of formula I:

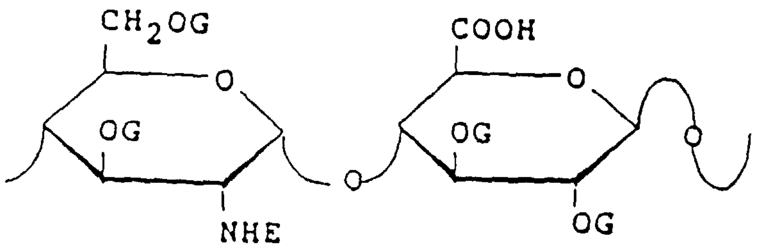

(I)

wherein

E represents an acetyl group, in 0 to 80% of the disaccharide units of said N,O-sulfated heparosanes, and a sulfate group and optionally a hydrogen atom in the remaining disaccharide units,
G represents a hydrogen atom and a sulfate group,
the degree of sulfation expressed as a ratio of sulfate to carboxyl being from 1.5 to 3.0,
the two ends, reducing and non-reducing, of the chains of said N,O-sulfated heparosanes being uronic units, which may or may not be sulfated, glucosamine, which may or may not be sulfated, or N-acetyl glucosamine, which may or may not be sulfated,
and the pharmaceutically acceptable salts of said N,O-sulfated heparosanes.

**4.** N,O-sulfated heparosanes according to any one of claims 1 to 3, characterised in that E represents an acetyl group in 0 to 60% of the disaccharide units of said N,O-sulfated heparosanes.

**5.** N,O-sulfated heparosanes according to any one of claims 1 to 4, characterised in that they comprise at least 90% by mass of chains having a molecular mass less than 11000 Da.

**6.** N,O-sulfated heparosanes according to claim 1, characterised in that they contain less than 0.2 µmol/mg of amino group ($NH_2$) .

**7.** N,O-sulfated heparosanes according to any one of claims 1 to 4, comprising chains having a mean molecular mass of 4000 Da to 7000 Da and having a degree of sulfation, expressed as the ratio of sulfate to carboxyl, of between 1.7 and 3.

**8.** N,O-sulfated heparosanes according to claim 1, characterised in that they comprise at least 70% by mass of chains having a molecular mass of between 5000 and 7000 Da, the degree of sulfation expressed as the ratio of sulfate to carboxyl is between 1.8 and 2.5, and E represents an acetyl group in 0 to 20% of the disaccharide units of said N,O-sulfated heparosanes.

**9.** N,O-sulfated heparosanes according to claim 1, characterised in that they comprise at least 70% by mass of chains having a molecular mass of between 10000 12000 Da, the degree of sulfation expressed as the ratio of sulfate to carboxyl is between 1.8 and 2.5, and E represents an acetyl group in 0 to 20% of the disaccharide units of said N,O-sulfated heparosanes.

**10.** N,O-sulfated heparosanes according to claim 1, characterised in that they comprise at least 70% by mass of chains having a molecular mass of between 6000 and 8000 Da, the degree of sulfation expressed as the ratio of sulfate to carboxyl is between 2.0 and 2.8, and E represents an acetyl group in 0 to 60% of the disaccharide units of said N,O-sulfated heparosanes.

**11.** N,O-sulfated heparosanes according to claim 1, characterised in that they comprise at least 80% by mass of chains having a molecular mass of between 2300 and 7200 Da, the degree of sulfation expressed as the ratio of sulfate to carboxyl is between 1.8 and 2.5, and E represents an acetyl group in 0 to 20% of the disaccharide units of said N,O-sulfated heparosanes

**12.** N,O-sulfated heparosanes according to claim 1, characterised in that they comprise at least 80% by mass of chains having a molecular mass of between 3300 and 7700 Da, the degree of sulfation expressed as the ratio of sulfate

to carboxyl is between 1.8 and 2.5, and E represents an acetyl group in 0 to 20% of the disaccharide units of said N,O-sulfated heparosanes.

**13.** N,O-sulfated heparosanes according to claim 1, characterised in that they comprise at least 70% by mass of chains having a molecular mass of between 6900 and 13500 Da, the degree of sulfation expressed as the ratio of sulfate to carboxyl is between 1.8 and 2.5, and E represents an acetyl group in 0 to 20% of the disaccharide units of said N,O-sulfated heparosanes.

**14.** N,O-sulfated heparosanes according to claim 1, characterised in that they comprise at least 80% by mass of chains having a molecular mass of between 4000 and 10300 Da, the degree of sulfation expressed as the ratio of sulfate to carboxyl is between 2.0 and 2.8, and E represents an acetyl group in 0 to 60% of the disaccharide units of said N,O-sulfated heparosanes.

**15.** An N,O-sulfated heparosane composition, characterised in that it contains at least 70% by mass of an N,O-sulfated heparosane according to any one of claims 1 to 14.

**16.** A process for the preparation of a composition containing 70% to 100% of an N,O-sulfated heparosane according to claim 1, characterised in that it comprises the sequence of steps as follows:

step a: cultivation of a strain of Escherichia coli (K5),

step b: isolation and purification, which may or may not be preceded by preliminary purification, of the N-acetylheparosane formed in order to obtain a composition containing 70% to 100% of an N-acetylheparosane comprising chains or a mixture of chains having a molecular mass of between 1500 and 15000 Da, having the repeating disaccharide structure of formula II

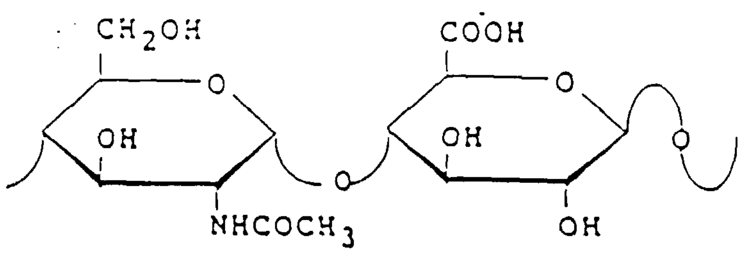

(II)

step c: partial deacetylation of this N-acetylheparosane composition in order to obtain a composition containing 70% to 100% of a heparosane comprising chains or a mixture of chains having a molecular mass of between 1500 and 15000 Da, having the repeating disaccharide structure of formula III

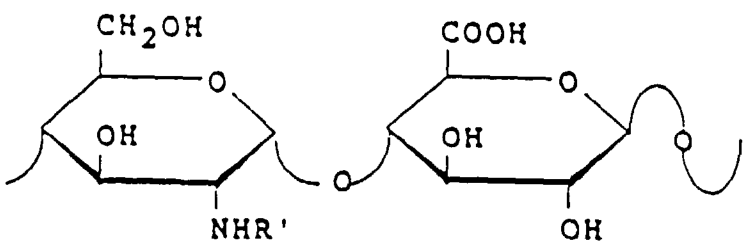

(III)

wherein R' represents an acetyl group in 0 to 80% of the disaccharide units and a hydrogen atom in the remaining disaccharide units,

step d:

- either partial N,O-sulfation of this heparosane composition,
- or partial N,O-sulfation of this heparosane composition followed by a step of total N-sulfation,
- or total or partial N-sulfation followed by a step of total or partial O-sulfation,

and optionally comprises one or more steps of fractionation of the molecular masses carried out at the end of step a, b, c or d.

**17.** A process according to claim 16, characterised in that the strain of Escherichia coli (K5) is the SEBR 3282 strain (deposited at the CNCM of the Pasteur Institute, Paris, France, under No. 1-1013) or a mutant of this strain, either spontaneous or induced.

**18.** A process according to claim 16, characterised in that the culturing of the Escherichia coli strain (K5) is continued at least two hours after the growth of the biomass has stopped.

**19.** A process according to claim 16, characterised in that the isolation and purification of the N-acetylheparosane comprise at least one step of alcohol precipitation and at least one step of ion exchange chromatography.

**20.** A process according to claim 16, characterised in that the isolation and purification of the N-acetylheparosane are carried out by means of a process comprising the sequence of steps as follows:

step $a_1$: precipitation with ethanol,
step $b_1$: dialysis,
step $c_1$: precipitation with ethanol followed by dehydration and drying,
step $d_1$ : purification by anion exchange chromatography,
step $e_1$: precipitation with ethanol of the eluate obtained in step $d_1$, dehydration, drying and grinding,

wherein one of steps $a_1$ or $c_1$ may be omitted.

**21.** A process according to claim 16, characterised in that the isolation and purification of the N-acetylheparosane are carried out using a process comprising the sequence of steps as follows:

step $a'_1$: dialysis,
step $b'_1$: purification in acidic medium, elimination of impurities which are insoluble in aqueous solutions of pH 3.5 and pH 1.8
step $c'_1$: precipitation with ethanol followed by dehydration and drying,
step $d'_1$: alkaline hydrolysis and dialysis,
step $e'_1$: purification by anion exchange chromatography,
step $f'_1$: purification by exclusion chromatography.

**22.** A process according to claim 16, characterised in that the N-acetylheparosane obtained by culturing a strain of Escherichia coli (K5) is subjected, before isolation and purification, to preliminary purification carried out by means of a process comprising the sequence of steps as follows:

step $a''_1$: centrifugation of the suspension obtained at the end of the culture,
step $b''_1$: contacting the supernatant with an alkaline solution,
step $c''_1$: preliminary filtration,
step $d''_1$: concentration on a membrane having a predetermined cut-off threshold,
step $e''_1$: dialysis,

of which step $e''_1$ may be omitted.

**23.** A process according to claim 16, characterised in that the step of partial N,O-sulfation is followed by total N-sulfation carried out using a complex of sulfuric anhydride with an organic base in an aqueous solvent of basic pH.

**24.** A process according to claim 16, characterised in that the step of total N-sulfation is followed by fractionation of the molecular masses.

**25.** A pharmaceutical composition containing as active principle an N,O-sulfated heparosane according to any one of claims 1 to 14, combined with or in admixture with a pharmaceutically acceptable inert excipient.

**26.** A pharmaceutical composition containing as active principle an N,O-sulfated heparosane composition according to claim 15, combined with or in admixture with a pharmaceutically acceptable inert excipient.

**27.** A pharmaceutical composition according to any one of claims 25 and 26 which may be used to regulate coagulation.

**28.** Heparosanes comprising a mixture of chains having a molecular mass of between 1500 and 15000 Da, having the repeating disaccharide structure of formula III:

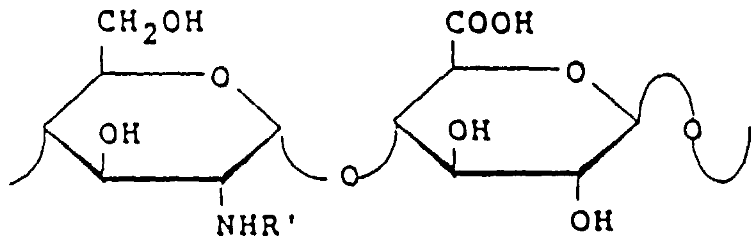

(III)

**29.** Heparosanes according to claim 28, characterised in that R' represents an acetyl group in 0 to 60% of the disaccharides units of said heparosanes.

**30.** A heparosane composition, characterised in that it contains at least 70% by mass of a heparosane according to either of claims 28 and 29.

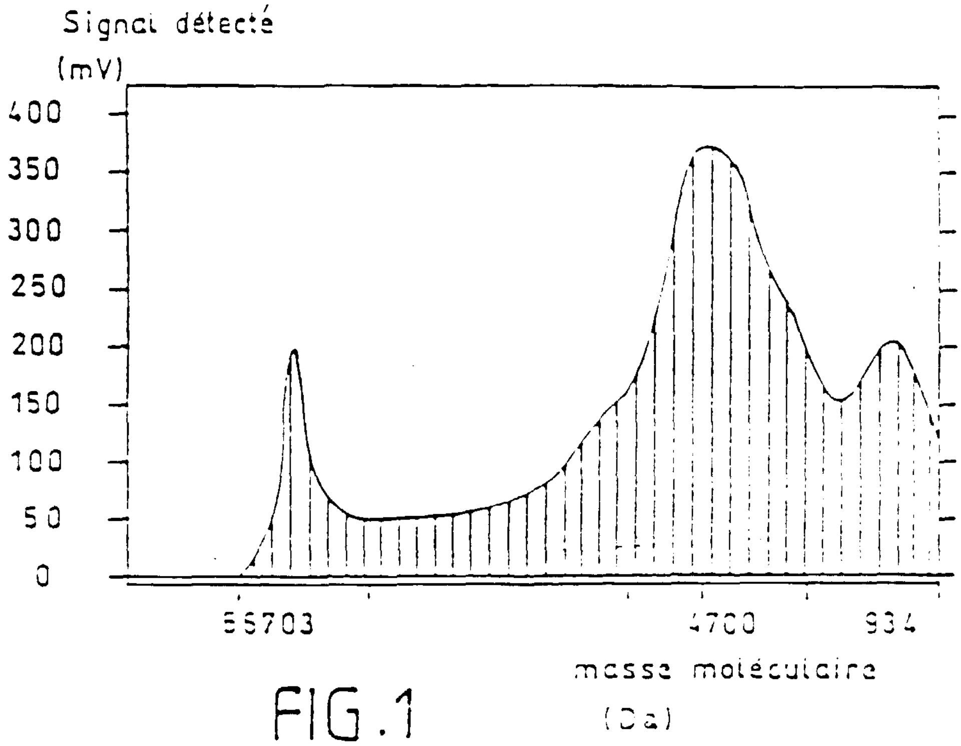
Signal détecté
(mV)
400
350
300
250
200
150
100
50
0
56703
4700
934
masse moléculaire
(Da)

FIG.1

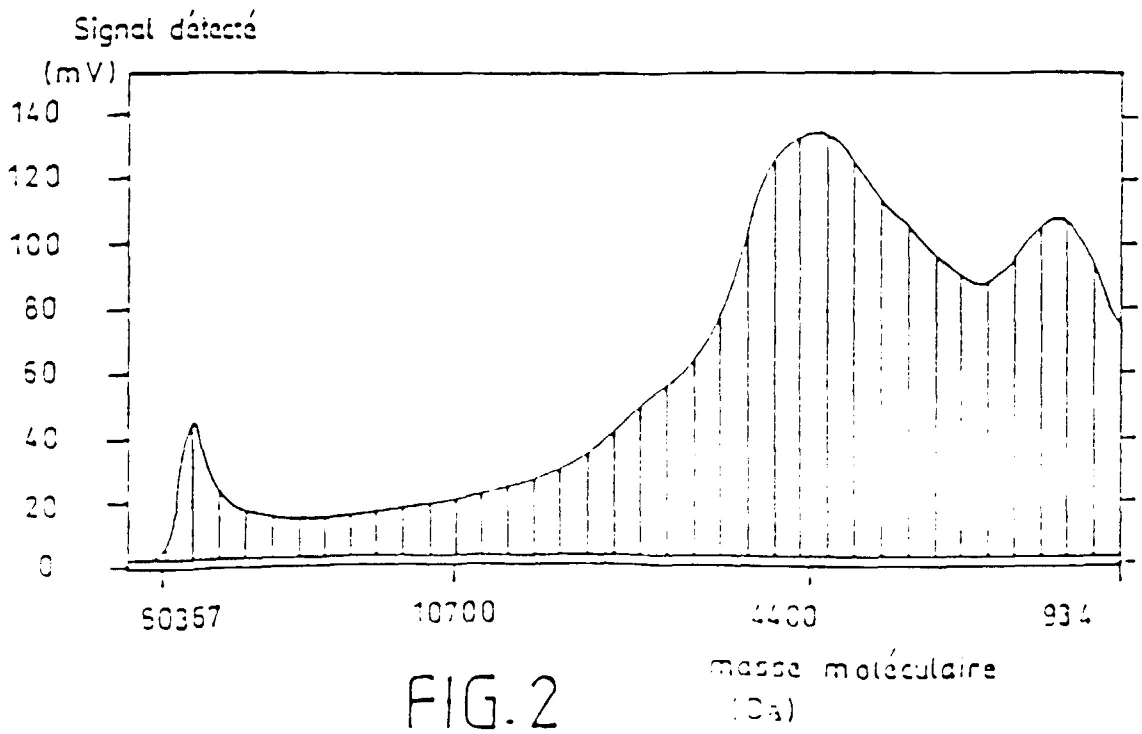
Signal détecté
(mV)
140
120
100
80
60
40
20
0
50367
10700
4400
934
masse moléculaire
(Da)

FIG.2

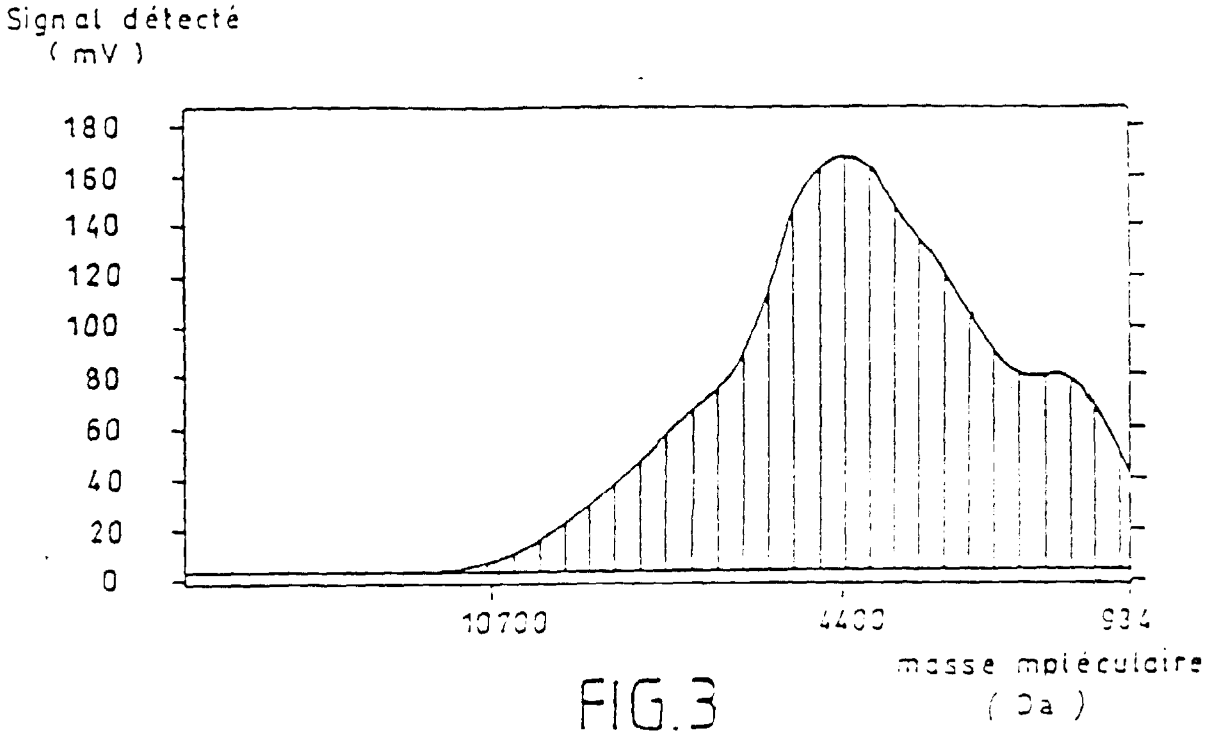
Signal détecté
( mV )
180
160
140
120
100
80
60
40
20
0
10700
4400
934
masse moléculaire
( Da )

FIG.3

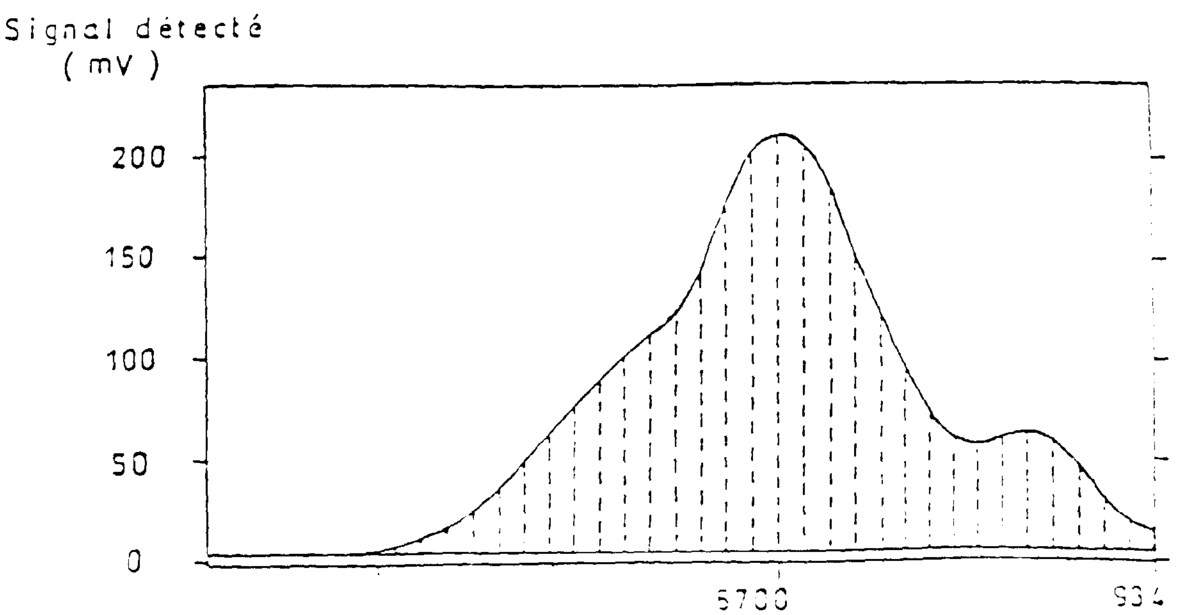
Signal détecté
( mV )
200
150
100
50
0
5700
934
masse moléculaire
( Da )

FIG.4

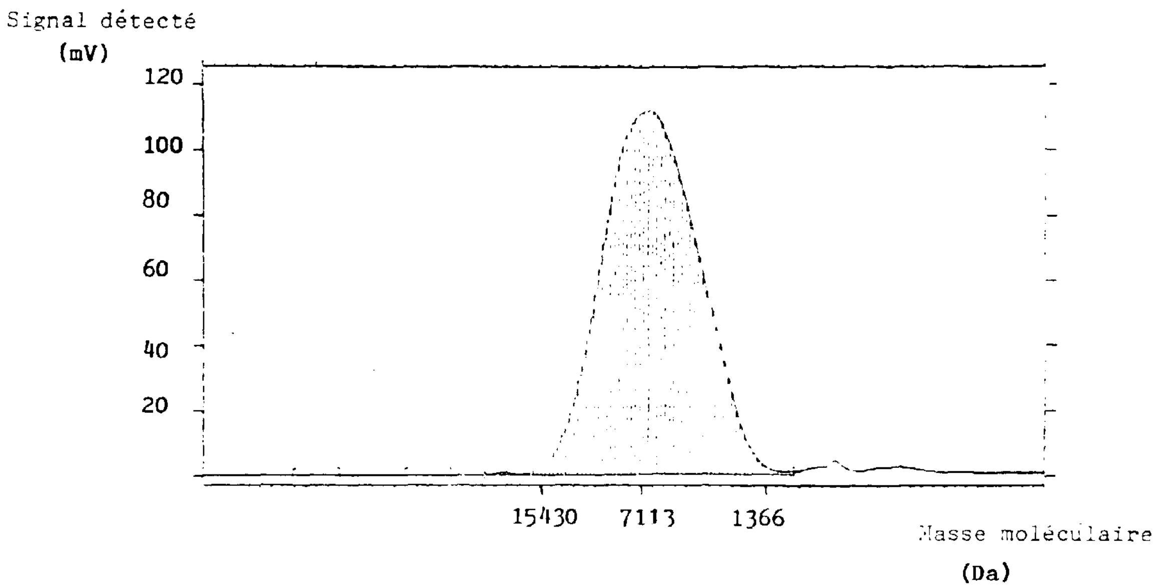
Signal détecté
(mV)
120
100
80
60
40
20
15430
7113
1366
Masse moléculaire
(Da)

FIG. 5